(19) **Europäisches Patentamt**
**European Patent Office**
**Office européen des brevets**

(11) **EP 3 129 505 B1**

(12) **EUROPEAN PATENT SPECIFICATION**

(45) Date of publication and mention
of the grant of the patent:
**30.06.2021 Bulletin 2021/26**

(21) Application number: **15776462.2**

(22) Date of filing: **11.04.2015**

(51) Int Cl.:
**C12Q 1/6844** (2018.01)

(86) International application number:
**PCT/US2015/025492**

(87) International publication number:
**WO 2015/157747 (15.10.2015 Gazette 2015/41)**

(54) **METHODS FOR CLONAL REPLICATION AND AMPLIFICATION OF NUCLEIC ACID MOLECULES FOR GENOMIC AND THERAPEUTIC APPLICATIONS**

VERFAHREN ZUR KLONALEN REPLIKATION UND AMPLIFIKATION VON NUKLEINSÄUREMOLEKÜLEN FÜR GENOMISCHE UND THERAPEUTISCHE ANWENDUNGEN

PROCEDES DE REPLICATION CLONALE ET D'AMPLIFICATION DE MOLECULES D'ACIDE NUCLEIQUE POUR DES APPLICATIONS GENOMIQUES ET THERAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.04.2014 US 201461978823 P**

(43) Date of publication of application:
**15.02.2017 Bulletin 2017/07**

(73) Proprietor: **RedVault Biosciences LP
Houston, TX 77027 (US)**

(72) Inventors:
• **METZKER, Michael, L.
Houston, TX 77024 (US)**
• **WEIER, Christopher, August
Houston, TX 77027 (US)**

(74) Representative: **Potter Clarkson
The Belgrave Centre
Talbot Street
Nottingham NG1 5GG (GB)**

(56) References cited:
EP-A1- 2 327 782    US-A1- 2002 012 930
US-A1- 2003 108 902    US-A1- 2003 108 902
US-A1- 2005 142 559    US-A1- 2007 141 610
US-A1- 2008 021 205    US-A1- 2008 153 763
US-A1- 2011 269 193    US-A1- 2011 269 193

• **GIRISH NALLUR ET AL: "Signal amplification by rolling circle amplification on DNA microarrays", NUCLEIC ACIDS RESEARCH, INFORMATION RETRIEVAL LTD, vol. 29, no. 23, December 2001 (2001-12), pages 1-9, XP008154013, ISSN: 0305-1048, DOI: 10.1093/NAR/29.23.E118**
• **ZHOU ET AL.: 'A dumbbell probe-mediated rolling circle amplification strategy for highly sensitive microRNA detection' NUCLEIC ACIDS RESEARCH vol. 38, no. 15, 2010, pages 1 - 5, XP055213312**
• **Valentin N Rybchin ET AL: "The plasmid prophage N15: a linear DNA with covalently closed ends", Molecular Microbiology, vol. 33, no. 5, 1 January 1999 (1999-01-01), pages 895-903, XP055607389,**
• **Seiyu Harada ET AL: "Episomal High Copy Number Maintenance of Hairpin-capped DNA Bearing a Replication Initiation Region in Human Cells", Journal of Biological Chemistry, vol. 284, no. 36, 4 September 2009 (2009-09-04), pages 24320-24327, XP055607431, US ISSN: 0021-9258, DOI: 10.1074/jbc.M109.008128**

## Description

### Field of the Invention

Embodiments of the present invention relate generally to the field of replication and amplification of nucleic acid molecules. More specifically, certain embodiments of the present invention involve the replication of DNA molecules from a biological sample using rolling circle replication. Other embodiments of the present invention involve the amplification of DNA molecules from a biological sample using rolling circle amplification. Certain embodiments of the invention may be utilized in the characterization of sequence variation in genomes derived from a biological sample. Certain embodiments of invention may be utilized in molecular counting of whole chromosomes or portions thereof derived from a biological sample. Certain embodiments of the invention may be utilized in the characterization of haplotype structure in genomes derived from a biological sample. Certain embodiments of invention may be applied for sample preparation and analysis in genomic sciences, biomedical research, diagnostic assays, and vaccine and therapeutic developments.

### Description of Related Art

Whole genome technologies, such as high-density genotyping arrays and next-generation sequencing (NGS), can identify sequence variation, particularly single nucleotide polymorphisms (SNPs) and single nucleotide variants (SNVs), collectively referred to herein as "sequence variants" of a given individual or species. Current methods, however, are unable to determine the combination of those sequence variants on the same DNA molecule. Determining the combination of sequence variants is termed "phase" and the specific combination of sequence variants on the same DNA molecule is termed a "haplotype." For example, human individuals are diploid, with each somatic cell containing two sets of autosomes that are inherited from each parent. Characterizing the haplotype status of a given individual is important for mapping disease genes, elucidating population histories, and studying the balance of *cis*- and *trans*-acting variants in phenotypic expression.

There are three general approaches to determining haplotype information: (*i*) population inference, (*ii*) parental inference, and (*iii*) molecular haplotyping. The most common approach for phasing haplotypes is using inference and statistical methods from data obtained from population or parental genotypes. Haplotype information across the entire genome, however, cannot be resolved using computational methods, particularly when linkage disequilibrium for a given chromosomal region is low and for rare variants. Parental inference methods, on the other hand, rely on the principles of genetic inheritance of sequence variation in the context of a family pedigree. While powerful when performed properly, many biological samples lack sufficient pedigree information or require appropriate family samples to infer the haplotype status of a given sample of interest.

Several molecular haplotyping methods are known to overcome the limitations of computationally-based approaches. These molecular methods include various strategies to isolate individual or sets of individual DNA molecules that are then genotyped or sequenced to determine the haplotype structure of a given biological sample. One such strategy involves the construction of large-insert clones (*i.e.,* fosmids) libraries. These clones are then diluted into individual wells of a multi-well plate (*i.e.,* 96- or 384-well plates), created into template libraries, barcoded to trace particular clones to individual wells, and characterized by genotyping or sequencing methods.

The challenge of phasing haplotypes of individual chromosomes or portions thereof becomes reduced to characterizing smaller DNA fragments (*i.e.,* from several hundred megabases to tens-to-hundreds of kilobases in size) in the diluted pools within the microtiter plates. Sizing DNA fragments or using genomic DNA in lieu of creating large-insert clones has also been reported followed by diluting, amplifying by whole-genome methods, creating template libraries, and sequencing to determine the haplotype of a given sample. Whole chromosomes or portions thereof can also be isolated by flow sorting methods or microdissection approaches, followed by diluting, amplifying by whole-genome methods, creating template libraries, and genotyping or sequencing to determine the haplotype structure of a given genome from a biological sample. All of these approaches require a high-level of technical expertise and the creation of large numbers of individual template libraries (on the order of hundreds) in phasing haplotypes of a given biological sample.

Most imaging systems cannot detect single fluorescent events, so DNA molecules in a sample have to be amplified. Three next-generation sequencing methods currently exist: (*i*) emulsion PCR (emPCR), (*ii*) solid-phase amplification, and (*iii*) solution-based rolling circle replication. For all these methods, genomic DNA is typically fragmented using standard physical shearing techniques to create a library of DNA fragments. There are exceptions where fragmenting may not be necessary. For example, some biological sources such as plasma or serum obtained from cancer patients or pregnant females contain circulating, cell-free genomic DNA fragments that typically exist in sizes under 1,000 base-pairs (bp) and in some cases under 500 bp. Depending upon whether an intervening step of size selection is needed, adapter sequences containing universal priming sites are then ligated to the DNA fragment ends. Limited number of PCR cycles are performed using common PCR primers. The three methods deviate at this step, but in all cases, these clonally-amplifying methods are limited to replicating or amplifying small fragments that are typically less than 1,000 bp in size, and in more typical examples, limited to 700 bp or less. For example, Illumina's method of solid-phase amplification can at best amplify DNA fragments that are only 700 bp in size. This size constraint limits the ability to assemble human genome de novo.

A significant drawback of current whole genome technologies, particularly NGS, is the reliance on sequence reads derived from short template libraries which are then clonally amplified in a massively parallel format. Importantly, current paired-end library construction methods inherently destroy the ability to easily identify large complex structural alterations that are present among normal human genomes and seem to be particularly important in the development of many diseases. Genomic structural variation may represent a driving force in early oncogenesis and cancer progression, disease susceptibility, and therapeutic resistance. Sequence reads derived from short template libraries make it exceedingly difficult to fully resolve novel, repetitive, and disease-altered sequences through de novo assembly. As such, most whole genome sequencing efforts still rely on the alignment of sequence reads to a reference genome. Consequently, NGS data sets may contain large stretches of the human genome sequence that remain uncharacterized, and understanding of disease mechanisms may be biased by a lack of genomic structural information.

Most whole genome sequencing efforts still rely on aligning sequence reads to a reference genome. While alignment experiments can capture a significant fraction of sequence variants, large templates on the order of 10-to-100 kb are needed to resolve a large portion of structural variants and/or to provide the phase of haplotypes across the human genome. A number of molecular biology and computer software techniques have been employed to overcome the size constraint. Despite providing some improvement, the trade-off is a significant increase in the complexity of the biological work-flow and cost associated with reagents, labor, and computer hardware.

Creating a DNA circle by ligating the ends of a linear nucleic acid fragment is a highly inefficient process, requiring a significant amount of starting material from a biological sample. The problems associated with creating circles by bringing distant ends of a given DNA fragment into close proximity to one another has been well established in the art since the 1980s. For example, one problem associated with creating circles by ligating the ends of a DNA fragment together is the competition reaction between "intramolecular" ligation events (i.e., DNA circles of the same DNA fragment) and "intermolecular" ligation events (i.e., joining of two or more DNA fragments called concatemers). Another problem associated with creating circles by ligating the ends of a DNA fragment together is that larger DNA fragments must be further diluted compared with smaller DNA fragments in order to achieve a reasonable efficiency in creating intramolecular circles.

US 2003/108902 A1 is entitled "Generation of single-strand circular DNA from linear self-annealing segments" and relates to a method in which two hairpin structures are ligated directly to each other, creating a particularly short double-stranded region in a dumbbell stem; this is exemplified in nucleic acid sequences of 45 and 50 nucleotides in length (paragraph [0073]). US 2011/269193 A1 is entitled "Method of amplifying a target nucleic acid by rolling circle amplification" and exemplifies its method with a 52-nucleic acid double-stranded sequence (paragraph [0046]). US 2002/012930 A1 is entitled "Method of sequencing a nucleic acid" and describes a method to permit a very large number of independent sequencing reactions to be arrayed in parallel, permitting simultaneous sequencing of a very large number (>10,000) of different oligonucleotides; it exemplifies the use of inserts of approximately only 65-75 nucleotides in length to create circular templates in the hundred nucleotide size range (Example 2, paragraphs 221-222), and an 88-nucleic acid single-stranded sequence that was used in an rolling circle amplification ("RCA") technique (Example 4, paragraph [0226]).

There is a need in the art for innovative methods that combine creating large DNA circles (i.e., the large-insert clones used in Sanger sequencing, which are 5-7-kb or larger) with the high-throughput replication or amplification nature of next-generation sequencing methods. Certain embodiments of the present invention overcome the size constraints of creating DNA circles from large DNA fragments by creating DNA circles in a size-independent manner. Other embodiments of the present invention overcome the size constraints of amplifying templates >5 kilobase directly by incorporating the size-independent DNA circles, by the creation and replication or amplification of large-insert templates useful in a number of genomic science applications. The present invention also overcomes the complexity of researcher efforts and associated higher costs of current methods by providing a simpler workflow for the preparation of large-insert templates using dumbbell circles and improved methods in rolling circle replication and rolling circle amplification to create multiple copies for sequencing applications. Certain embodiments of the invention also overcome the limitation of requiring individual allele-discriminating primers for genotyping and sequencing applications of a diverse set of heterogeneous nucleic acid sequences by providing a simpler workflow for the preparation templates that rely on universal primer sequences.

## SUMMARY

The present invention is defined by the claims.

More particularly, the invention provides a method of replication or amplification of at least one nucleic acid molecule, the method comprising: (a) ligating hairpin structures to each end of at least one double-stranded nucleic acid molecule with a minimum length of greater than 5 kb isolated from a sample to form at least one dumbbell template; (b) contacting said at least one dumbbell template with at least one substantially complementary primer in a method of replication or with at least two substantially complementary primers in a method of amplification, wherein said at least one substantially complementary primer is attached to at least one substrate; and (c) performing rolling circle replication on said at least one dumbbell template contacted with the at least one substantially complementary primer to form at least one replicated or amplified dumbbell template.

One embodiment of the invention is a method of replication of at least one nucleic acid (e.g. DNA) molecule. The method may include the steps of fragmenting at least one nucleic acid (e.g. DNA) molecule to form at least one fragmented double-stranded nucleic acid (e.g. DNA) molecule with a minimum length of greater than 5 kb, greater than 10 kb, or at least 20 kb long; ligating hairpin structures to each end of the at least one fragmented nucleic acid (e.g. DNA) molecule to form at least one dumbbell template; contacting the at least one dumbbell template with at least one substantially complementary primer, wherein the at least one substantially complementary primer is attached to at least one substrate; and performing rolling circle replication on the at least one dumbbell template contacted with the at least one substantially complementary primer to form at least one replicated dumbbell template.

Another embodiment of the invention is a method of replication of at least one nucleic acid (e.g. DNA) molecule. The method may include the steps of fragmenting at least one nucleic acid (e.g. DNA) molecule to form at least one fragmented double-stranded nucleic acid (e.g. DNA) molecule with a minimum length of greater than 5 kb, greater than 10 kb, or at least 20 kb; ligating hairpin structures to each end of the at least one fragmented nucleic acid (e.g. DNA) molecule to form at least one dumbbell template; purifying the at least one dumbbell template by treating any unligated hairpin structure and any unligated fragmented nucleic acid molecule with an exonuclease; contacting the at least one dumbbell template with at least one substantially complementary primer, wherein the at least one substantially complementary primer is attached to at least one substrate; and performing rolling circle replication on the at least one dumbbell template contacted with the at least one substantially complementary primer to form at least one replicated dumbbell template.

Another embodiment of the invention is a method of amplification of at least one nucleic acid (e.g. DNA) molecule. The method may include the steps of fragmenting at least one nucleic acid (e.g. DNA) molecule to form at least one fragmented double-stranded nucleic acid (e.g. DNA) molecule with a minimum length of greater than 5 kb, greater than 10 kb or at least 20 kb; ligating hairpin structures to each end of the at least one fragmented DNA molecule to form at least one dumbbell template; contacting the at least one dumbbell template with at least two substantially complementary primer, wherein at least one substantially complementary primer is attached to at least one substrate; and performing rolling circle amplification on the at least one dumbbell template contacted with the at least one substantially complementary primer to form at least one replicated dumbbell template.

Another embodiment of the invention is a method of detecting at least one replicated dumbbell template, the method comprising: (a) fragmenting at least one nucleic acid molecule to form at least one fragmented nucleic acid molecule with a minimum length of greater than 5 kb, greater than 10 kb or at least 20 kilobases long; (b) replicating the fragmented nucleic acid molecule according to the replication method of paragraph [0013]; and (c) detecting said at least one replicated dumbbell template. Optionally, the method includes the steps of fragmenting at least one nucleic acid (e.g. DNA) molecule to form at least one fragmented double-stranded nucleic acid (e.g. DNA) molecule with a minimum length of greater than 5 kb, greater than 10 kb, or at least 20 kb; ligating hairpin structures to each end of the at least one fragmented nucleic acid (e.g. DNA) molecule to form at least one dumbbell template; contacting the at least one dumbbell template with at least one substantially complementary primer, wherein the at least one substantially complementary primer is attached to at least one substrate; performing rolling circle replication on the at least one dumbbell template contacted with the at least one substantially complementary primer to form at least one replicated dumbbell template; and detecting the at least one replicated dumbbell template. In another embodiment, the step of detecting the at least one replicated dumbbell template consists of sequencing the at least one replicated dumbbell template.

Another embodiment of the invention is a method of detecting at least one replicated dumbbell template. The method includes the steps of fragmenting at least one nucleic acid (e.g. DNA) molecule to form at least one fragmented double-stranded nucleic acid (e.g. DNA) molecule with a minimum length of greater than 5 kb, at least 10 kb, or greater than 20 kb; ligating hairpin structures to each end of the at least one fragmented nucleic acid (e.g. DNA) molecule to form at least one dumbbell template; purifying the at least one dumbbell template by treating any unligated hairpin structure and any unligated fragmented nucleic acid molecule with an exonuclease; contacting the at least one dumbbell template with at least one substantially complementary primer, wherein the at least one substantially complementary primer is attached to at least one substrate; performing rolling circle replication on the at least one dumbbell template contacted with the at least one substantially complementary primer to form at least one replicated dumbbell template; and detecting the at least one replicated dumbbell template. In another embodiment, the step of detecting the at least one replicated dumbbell template consists of sequencing the at least one replicated dumbbell template.

In certain embodiments, the step of detecting the at least one replicated dumbbell template includes contacting said at least one replicated dumbbell template with an oligonucleotide probe. In certain embodiments, the oligonucleotide probe is a labeled oligonucleotide probe. In certain embodiments, the oligonucleotide probe is a labeled DNA probe. In certain embodiments, the oligonucleotide probe is attached to a fluorophore.

Another embodiment of the invention is a method of detecting at least one amplified dumbbell template, the method comprising: (a) fragmenting at least one nucleic acid molecule to form at least one fragmented nucleic acid molecule with a minimum length of greater than 5 kb, greater than 10 kb or at least 20 kilobases long; (b) amplifying the fragmented nucleic acid molecule according to the amplification method of paragraph [0013]; and (c) detecting said at least one amplified dumbbell template. The method may include the steps of fragmenting at least one nucleic acid (e.g. DNA) molecule to form at least one fragmented double-stranded nucleic acid (e.g. DNA) molecule with a minimum length of greater than 5 kb, greater than 10 kb, or at least 20 kb; ligating hairpin structures to each end of the at least one fragmented DNA molecule to form at least one dumbbell template; contacting the at least one dumbbell template with at least two substantially complementary primer, wherein at least one substantially complementary primer is attached to at least one substrate; performing rolling circle amplification on the at least one dumbbell template contacted with the at least one substantially complementary primer to form at least one amplified nucleic acid (e.g. DNA) molecule; and detecting the at least one amplified nucleic acid (e.g. DNA) molecule. In another embodiment, the step of detecting the at least one amplified nucleic acid (e.g. DNA) molecule consists of sequencing the at least one amplified nucleic acid (e.g. DNA) molecule.

In certain embodiments, the step of detecting the at least one amplified dumbbell template includes contacting said at least one amplified dumbbell template with an oligonucleotide probe. In certain embodiments, the oligonucleotide probe is a labeled oligonucleotide probe. In certain embodiments, the oligonucleotide probe is a labeled DNA probe. In certain embodiments, the oligonucleotide probe is attached to a fluorophore.

Another embodiment of the invention is a method of replication of at least one nucleic acid (e.g. DNA) molecule. The method includes the steps of isolating at least one nucleic acid (e.g. DNA) molecule from a sample; fragmenting at least one nucleic acid (e.g. DNA) molecule to form at least one fragmented double-stranded nucleic acid (e.g. DNA) molecule with a minimum length of greater than 5 kb, greater than 10 kb, or at least 20 kb; ligating hairpin structures to each end of the at least one fragmented nucleic acid (e.g. DNA) molecule to form at least one dumbbell template; contacting the at least one dumbbell template with at least one substantially complementary primer, wherein the at least one substantially complementary primer is attached to at least one substrate; and performing rolling circle replication on the at least one dumbbell template contacted with the at least one substantially complementary primer to form at least one replicated dumbbell template.

Another embodiment of the invention is a method of amplification of at least one nucleic acid (e.g. DNA) molecule. The method includes the steps of isolating at least one DNA molecule from a sample; fragmenting at least one DNA molecule to form at least one fragmented double-stranded DNA molecule with a minimum length of greater than 5 kb; ligating hairpin structures to each end of the at least one fragmented DNA molecule to form at least one dumbbell template; contacting the at least one dumbbell template with at least two substantially complementary primer, wherein the at least one substantially complementary primer is attached to at least one substrate; and performing rolling circle amplification on the at least one dumbbell template contacted with the at least one substantially complementary primer to form at least one amplified nucleic acid (e.g. DNA) molecule.

Another embodiment of the invention is a method of replication of at least one nucleic acid (e.g. DNA) molecule. The method includes the steps of isolating at least one nucleic acid (e.g. DNA) molecule from a sample; ligating hairpin structures to each end of the at least one double-stranded nucleic acid (e.g. DNA) molecule with a minimum length of greater than 5 kb, greater than 10 kb, or at least 20 kb, to form at least one dumbbell template; contacting the at least one dumbbell template with at least one substantially complementary primer, wherein the at least one substantially complementary primer is attached to at least one substrate; and performing rolling circle replication on the at least one dumbbell template contacted with the at least one substantially complementary primer to form at least one replicated dumbbell template.

Another embodiment of the invention is a method of amplification of at least one nucleic acid (e.g. DNA) molecule. The method includes the steps of isolating at least one nucleic acid (e.g. DNA) molecule from a sample; ligating hairpin structures to each end of the at least one double stranded nucleic acid (e.g. DNA) molecule with a minimum length of greater than 5 kb, greater than 10 kb, or at least 20 kb, to form at least one dumbbell template; contacting the at least one dumbbell template with at least two substantially complementary primers, wherein at least one substantially complementary primer is attached to at least one substrate; and performing rolling circle amplification on the at least one dumbbell template contacted with the at least one substantially complementary primer to form at least one amplified nucleic acid (e.g. DNA) molecule.

Another embodiment of the invention is a method of detecting at least one amplified dumbbell template. The method includes fragmenting at least one nucleic acid molecule to form at least one fragmented double-stranded nucleic acid molecule with a minimum length of greater than 5 kb, greater than 10 kb, or at least 20 kb; ligating hairpin structures to each end of said at least one fragmented nucleic acid molecule to form at least one dumbbell template; purifying said at least one dumbbell template by treating any unligated hairpin structure and any unligated fragmented nucleic acid molecule with an exonuclease; contacting said at least one dumbbell template with at least two substantially complementary primers, wherein said at least one substantially complementary primer is attached to at least one substrate; performing rolling circle amplification on said at least one dumbbell template contacted with the at least one substantially complementary primer to form at least one amplified dumbbell template; and detecting said at least one amplified dumbbell template.

Another embodiment of the invention is a method of amplification of at least one nucleic acid molecule. The method includes isolating at least one nucleic acid molecule from a sample; ligating hairpin structures to each end of said at least one double-stranded nucleic acid molecule with a minimum length of greater than 5 kb, greater than 10 kb, or at least 20 kb, to form at least one dumbbell template; purifying said at least one dumbbell template by treating any unligated hairpin structure and any unligated fragmented nucleic acid molecule with an exonuclease; contacting said at least one dumbbell template with at least two substantially complementary primers, wherein said at least one substantially complementary primer is attached to at least one substrate; and performing rolling circle amplification on said at least one dumbbell template contacted with the at least one substantially complementary primer to form at least one amplified dumbbell template.

Disclosures of the present application further include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a polymerase and at least one primer substantially complementary to a region of the at least one dumbbell template for replicating the at least one dumbbell template to form at least one replicated dumbbell template.

Certain disclosures of the present application include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a replisome and at least one primer substantially complementary to a region of the at least one dumbbell template for replicating the at least one dumbbell template to form at least one replicated dumbbell template.

Certain disclosures of the present application include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a polymerase and at least two primers substantially complementary to at least two regions of the at least one dumbbell template for amplifying the at least one dumbbell template to form at least one amplified dumbbell template.

Certain disclosures of the present application include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a replisome and at least two primers substantially complementary to at least two regions of the at least one dumbbell template for amplifying the at least one dumbbell template to form at least one amplified dumbbell template.

## BRIEF DESCRIPTION OF THE DRAWINGS

So that the manner in which the features and benefits of the invention, as well as others which will become apparent, may be understood in more detail, a more particular description of the embodiments of the invention may be had by reference to the embodiments thereof which are illustrated in the appended drawings, which form a part of this specification. It is also to be noted, however, that the drawings illustrate only various embodiments of the invention and are therefore not to be considered limiting of the invention's scope as it may include other effective embodiments as well.

FIG. 1 is a schematic diagram of an exemplary method of rolling circle replication of a dumbbell template according to an embodiment of the invention.

FIG. 2 is an image of the agarose gel analysis of the rolling circle products produced from the dumbbell templates, according to an embodiment of the invention.

FIG. 3 a schematic diagram of an exemplary method of rolling circle replication of a dumbbell template according to an embodiment of the invention.

FIG. 4 is an image of the agarose gel analysis of the dumbbell templates and their rolling circle products produced according to an embodiment of the invention.

FIG. 5 is an image of the agarose gel analysis of the dumbbell templates and their rolling circle products produced according to an embodiment of the invention.

FIG. 6 is an image of the agarose gel analysis of the dumbbell templates produced according to an embodiment of the invention.

FIG. 7 is an image of the agarose gel analysis of the dumbbell templates produced according to an embodiment of the invention.

FIG. 8 is an image of the agarose gel analysis of the rolling circle products produced according to an embodiment of the invention.

FIG. 9 is an image of the agarose gel analysis of the rolling circle products produced according to an embodiment of the invention.

FIG. 10 is a graph demonstrating detection of hairpin structures by fluorescence, according to an embodiment of the invention.

FIG. 11A and 11B are images of an exemplary device according to certain embodiments of the invention.

## DETAILED DESCRIPTION

Before describing the embodiments of the present invention in detail, several terms used in the context of the embodiments of the present invention will be defined. In addition to these terms, others are defined elsewhere in the specification, as necessary. Unless otherwise expressly defined herein, terms of art used in this specification will have their art-recognized meanings.

To more readily facilitate an understanding of the invention, the meanings of terms used herein will become apparent from the context of this specification in view of common usage of various terms and the explicit definitions provided below. As used herein, the terms "comprise or comprising," "contain or containing," "include or including," and "such as" are used in their open, non-limiting sense.

"Amplified dumbbell template" means one nucleic acid molecule containing one or more hairpin structures that results in multiple copies of the target sequence as a result of rolling circle amplification.

"Contacting" means a process whereby a substance is introduced by any manner to promote an interaction with another substance. For example, and without limitation, a dumbbell template may be contacted with one or more substantially complementary primers to promote one or more hybridizing processes to form one or more double-stranded duplex regions capable of participating in rolling circle replication or rolling circle amplification.

"Detecting a nucleic acid molecule" means using an analytical method that can determine the presence of the nucleic acid of interest or that can determine more detailed information regarding the nucleic acid sequence, alterations of a nucleic acid sequence when compared with a reference sequence, or the presence or absence of one or more copies of the nucleic acid sequence.

"Dumbbell template" means a structurally linear, and topologically circular *in vitro* replication competent or *in vitro* amplification competent nucleic acid molecule that has one or more hairpin structures. When denatured or substantially denatured, dumbbell templates exist as circular, single-stranded nucleic acid molecules. Dumbbell templates are distinct from *in vivo* replication competent circular, double-stranded DNA, for example and without limitation, plasmids, cosmids, fosmids, bacterial artificial chromosomes, and yeast artificial chromosomes, which are created by the aid of cloning vector technologies. Unlike these circular, double-stranded DNAs that replicate independently in appropriate host cells, dumbbell templates do not require propagation replication in such host cells.

"End(s) of a fragmented nucleic acid molecule(s)" means one or more terminal nucleotide residues capable or to be made capable of participating in a ligation reaction. In certain embodiments, one or more nucleic acid molecules may contain functional ends capable or to be made capable of a ligation reaction to attach one or more hairpin structures to each end of the nucleic acid molecule. For example, and without limitation, the 5'-end terminal nucleotide contains a phosphate group and the 3'-end terminal nucleotide contains a hydroxyl group.

"Fragmented nucleic acid molecule" means any larger nucleic acid molecule that becomes any smaller nucleic acid molecule resulting from the fragmenting process.

"Fragmenting" means the breaking of nucleic acid molecules in a non-sequence-dependent manner (*i.e.,* randomly) or in a sequence-specific manner using chemical or biochemical agents. For example, nucleic acids can be randomly fragmented by enzymatic methods using DNase I, endonuclease V, or transposases, using physical methods, like shearing, sonication, or nebulation, the latter of which passes a nucleic acid solution through a small hole, or using mechanical forces, for example, and without limitation, acoustic methods and particularly adaptive focused acoustic methods. Random nucleic acid fragments can be made by PCR using random primers. Nucleic acids can also be fragmented by sequence-specific methods, for example and without limitation, using restriction endonucleases and multiplex PCR. The collection of fragments derived from the fragmenting process of a larger nucleic acid molecule or molecules is called a library.

"Hairpin structure" means a nucleic acid molecule whereby two or more partial sequences within the nucleic acid molecule are complementary or substantially complementary to each other resulting in the formation of a partially double-stranded region and one or more internal single-stranded regions. The hairpin structure can also contain two or more nucleic acid molecules whereby the two or more nucleic acid molecules are joined together by a linker and whereby two or more partial sequences of the two or more nucleic acid molecules are complementary or substantially complementary to each other resulting in the formation of a partially double-stranded region and one or more internal single-stranded regions.

"Isolating a nucleic acid molecule" means a process whereby a nucleic acid molecule is obtained from a sample.

"Ligating agents" means the covalent joining of two or more nucleic acid molecules by enzymatic agents, for example and without limitation, DNA or RNA ligase or chemical agents, for example and without limitation, condensation reactions using water soluble carbodiimide or cyanogen bromide as well as standard practices associated with automated DNA synthesis techniques, resulting in a natural nucleic acid backbone structure, modified nucleic acid backbone structure, and combination of the two backbone structures thereof. A natural nucleic acid backbone structure, for example and without limitation, consists of one or more standard phosphodiester linkages between nucleotide residues. A modified nucleic acid backbone structure, for example and without limitation, consists of one or more modified phosphodiester linkages such as substitution of the non-bridging oxygen atom with a nitrogen atom (*i.e.,* a phosphoramidate linkage or a sulfur atom (*i.e.,* a phosphorothioate linkage), substitution of the bridging oxygen atom with a sulfur atom (*i.e.,* phosphorothiolate), substitution of the phosphodiester bond with a peptide bond (*i.e.,* peptide nucleic acid or PNA), or formation of one or more additional covalent bonds (*i.e.,* locked nucleic acid or LNA), which has an additional bond between the 2'-oxygen and the 4'-carbon of the ribose sugar. The modified linkages may be of all one type of modification or any combination of two or more modification types and further may comprise one or more standard phosphodiester linkages.

"Linker" means one or more divalent groups (linking members) that function as a covalently-bonded molecular bridge between two other nucleic acid molecules. A linker may contain one or more linking members and one or more types of linking members. Exemplary linking members include: -C(O)NH-, -C(O)O-, -NH-, -S-,-S(O)$n$-where n is 0, 1, or 2, -O-, -OP(O)(OH)O-, -OP(O)(O$^-$)O-, alkanediyl, alkenediyl, alkynediyl, arenediyl, heteroarenediyl, or combinations thereof. Some linkers have pendant side chains or pendant functional groups (or both). Pendant moieties can be hydrophilicity modifiers (*i.e.,* chemical groups that increase the water solubility properties of the linker), for example and without limitation, solubilizing groups such as -SO$_3$H, -SO$_3^-$, CO$_2$H or CO$_2^-$.

"Nucleic acid molecule" means any single-stranded or double-stranded nucleic acid molecule including standard canonical bases, hypermodified bases, non-natural bases, or any combination of the bases thereof. For example and without limitation, the nucleic acid molecule contains the four canonical DNA bases - adenine, cytosine, guanine, and thymine, or the four canonical RNA bases - adenine, cytosine, guanine, and uracil. Uracil can be substituted for thymine when the nucleoside contains a 2'-deoxyribose group. The nucleic acid molecule can be transformed from RNA into DNA and from DNA into RNA. For example, and without limitation, mRNA can be created in complementary DNA (cDNA) using reverse transcriptase and DNA can be created into RNA using RNA polymerase. The nucleic acid molecule can also contain one or more hypermodified bases, for example and without limitation, 5-hydroxymethyluracil, 5-hydroxyuracil, $\alpha$-putrescinylthymine, 5-hydroxymethylcytosine, 5-hydroxycytosine, 5-methylcytosine, $N^4$-methylcytosine, 2-aminoadenine, $\alpha$-carbamoylmethyladenine, $N^6$-methyladenine, inosine, xanthine, hypoxanthine, 2,6-diaminpurine, and $N^7$-methylguanine. The nucleic acid molecule can also contain one or more non-natural bases, for example and without limitation, 7-deaza-7-hydroxymethyladenine, 7-deaza-7-hydroxymethylguanine, isocytosine (isoC), 5-methylisocytosine, and isoguanine (isoG). The nucleic acid molecule containing only canonical, hypermodified, non-natural bases, or any combinations the bases thereof, can also contain, for example and without limitation where each linkage between nucleotide residues can consist of a standard phosphodiester linkage, and in addition, may contain one or more modified linkages, for example and without limitation, substitution of the non-bridging oxygen atom with a nitrogen atom (*i.e.,* a phosphoramidate linkage, a sulfur atom (*i.e.,* a phosphorothioate linkage), or an alkyl or aryl group (*i.e.,* alkyl or aryl phosphonates), substitution of the bridging oxygen atom with a sulfur atom (*i.e.,* phosphorothiolate), substitution of the phosphodiester bond with a peptide bond (*i.e.,* peptide nucleic acid or PNA), or formation of one or more additional covalent bonds (*i.e.,* locked nucleic acid or LNA), which has an additional bond between the 2'-oxygen and the 4'-carbon of the ribose sugar. The term "2'-deoxyribonucleic acid molecule" means the same as the term "nucleic acid molecule" with the limitation that the 2'-carbon atom of the 2'-deoxyribose group contains at least one hydrogen atom. The term "ribonucleic acid molecule" means the same as the term "nucleic acid molecule" with the limitation that the 2'-carbon atom of the ribose group contains at least one hydroxyl group.

"Nucleic acid sequence" means the order of canonical bases, hypermodified bases, non-natural bases, or any combination of the bases thereof present in the nucleic acid molecule.

"Performing" means providing all necessary components, reagents, and conditions that enable a chemical or biochemical reaction to occur to obtain the desired product.

"Purifying" means separating substantially all the undesired components from the desired components of a given mixture. For example, without limitation, purifying dumbbell templates refers to a method of removing undesired nucleic acid molecules that did not successfully ligate to form dumbbell templates for any given size range.

"Replicated dumbbell template" means one nucleic acid molecule containing one or more hairpin structures that results in multiple copies of the target sequence as a result of rolling circle replication.

"Rolling circle amplification" or "RCA" means a biochemical process using two or more primers whereby the copied nucleic acid molecules in addition to the original dumbbell template serves as template in subsequent amplification rounds to make more copies of the starting nucleic acid molecule.

"Rolling circle replication" or "RCR" means a biochemical process using one or more primers whereby the copied nucleic acid molecules do not serve as template in subsequent replication rounds to make more copies of the starting nucleic acid molecule. In certain embodiments, when the dumbbell template is a plus strand, the rolling circle replication results in more copies of the minus strand. In certain embodiments, when the dumbbell template is a minus strand, the rolling circle replication results in more copies of the plus strand. As used herein replication is distinct from amplification, which utilizes the copied nucleic acid in subsequent amplification rounds to make more copies of the starting nucleic acid molecule.

"Sample" means a material obtained from a biological sample or synthetically-created source that contains a nucleic acid molecule of interest. In certain embodiments, a sample is the biological material that contains the desired nucleic acid for which data or information are sought. Samples can include at least one cell, fetal cell, cell culture, tissue specimen, blood, serum, plasma, saliva, urine, tear, vaginal secretion, sweat, lymph fluid, cerebrospinal fluid, mucosa secretion, peritoneal fluid, ascites fluid, fecal matter, body exudates, umbilical cord blood, chorionic villi, amniotic fluid, embryonic tissue, multicellular embryo, lysate, extract, solution, or reaction mixture suspected of containing a target nucleic acid molecule. Samples can also include non-human sources, such as non-human primates, rodents and other mammals, pathogenic species including viruses, bacteria, and fungi. In certain embodiments, the sample can also include isolations from environmental sources for the detection of human and non-human species as well as pathogenic species in blood, water, air, soil, food, and for the identification of all organisms in the sample without any prior knowledge. In certain embodiments, the sample may contain nucleic acid molecules that are degraded. Nucleic acid molecules can have nicks, breaks or modifications resulting from exposure to physical forces, such as shear forces, to harsh environments such as heat or ultraviolet light, to chemical degradation processes such as may be employed in clinical or forensic analyses, to biological degradation processes due to microorganisms or age, to purification or isolation techniques, or a combination thereof.

"Sequencing" means any biochemical method that can identify the order of nucleotides from a replicated dumbbell template or an amplified dumbbell template.

"Substantially complementary primer" means a nucleic acid molecule that forms a stable double-stranded duplex with another nucleic acid molecule, although one or more bases of the nucleic acid sequence within the duplex region do not base-pair(s) with the another nucleic acid sequence.

The basic structure of single-stranded and double-stranded nucleic acid molecules is dictated by base-pair interactions. For example, the formation of base-pairs between complementary or substantially complementary nucleotides on the two opposite strands will cause the two strands to coil around each other to form a double-helix structure. This is called intermolecular base-pairing of complementary nucleotides of two or more nucleic acid molecule strands. The term "nucleotide" is defined broadly in the present invention as a unit consisting of a sugar, base, and one or more phosphate groups, for which the sugar, for example, and without limitation, consists of a ribose, a modified ribose with additional chemical groups attached to one or more atoms of the ribose group, a 2'-deoxyribose, or a modified 2'-deoxyribose with additional chemical groups attached to one or more atoms of the 2'-deoxyribose group, and for which the base, for example, and without limitation, consists of a canonical base, hypermodified base, or non-natural base, as described in the nucleic acid molecule definition above. Base-pairing of complementary nucleotides or substantially complementary nucleotides can also occur on the same DNA strand molecule, called intramolecular base-pairing of complementary nucleotides or substantially complementary nucleotides.

Hairpin structures can be formed by intramolecular base-pairing of complementary nucleotides or substantially complementary nucleotides of a given nucleic acid molecule, which can form a stem-loop structure. The stem portion of the hairpin structure is formed by hybridization of the complementary nucleotide or substantially complementary nucleotide sequences to form a double-stranded helix stretch. The loop region of the hairpin structure is the result of an unpaired stretch of nucleotide sequences. The stability of the hairpin structure is dependent on the length, nucleic acid sequence composition, and degree of base-pair complementary or substantial complementary of the stem region. For example, a stretch of five complementary nucleotides may be considered more stable than a stretch of three complementary nucleotides or a stretch of complementary nucleotides that are predominately composed of guanines and cytosines may be considered more stable than a stretch of complementary nucleotides that are predominately composed of adenines and thymines (DNA) or uracils (RNA). Modified nucleotides may be substituted to alter the stability of the double-stranded stem region for these natural bases, examples of which include, but are not limited to, inosine, xanthine, hypoxanthine, 2,6-diaminpurine, $N^6$-methyladenine, 5-methylcytosine, 7-deazapurines, 5-hydroxylmethylpyrimidines. Modified nucleotides may also include numerous modified bases found in RNA species. Natural occurring stem-loop structures are predominately found in RNA species, such as transfer RNA (tRNA), pre-microRNA, ribozymes and their equivalents.

Nucleic acid hairpin structures may be generated by deliberate design using methods of manufacturing synthetic oligonucleotides. Oligonucleotides are widely used as primers for DNA sequencing and PCR, as probes for screening and detection experiments, and as linkers or adapters for cloning purposes. Short oligonucleotides in the range of 15 - 25 nucleotides can be used directly without purification. As the stepwise yields are less than 100%, longer oligonucleotides require purification by high performance liquid chromatography or HPLC, or by preparative gel electrophoresis to remove failed oligonucleotide fractions, also known as n-1, n-2, etc. products. In certain embodiments, the nucleic acid hairpin is approximately about 100 bases.

Depending on the nature of the experiment, a given hairpin structure may be designed to contain a desired stability of the double-stranded duplex by substituting one or more hypermodified or non-natural bases and/or one or more backbone linkages as discussed herein, or including other synthetic bases such as 7-deaza-7-hydroxypurines, isoC and isoG, or their equivalents, as well as creating, for example, and without limitation, RNA-DNA, PNA-DNA, PNA-RNA, PNA-PNA, LNA-DNA, LNA-RNA, LNA-LNA double-stranded duplexes. Synthetically-designed hairpin structures are useful in several molecular biology techniques, for example, and without limitation, as priming sites for DNA polymerase by ligating hairpins to the ends of DNA fragments, detecting moieties as probes to identify a sequence of interest, and creating topologically circular DNA molecules from linear fragments. In certain embodiments, the 5'-ends of hairpin structures will be phosphorylated, for example and without limitation, using $T_4$ polynucleotide kinase to facilitate the efficient ligation using ligating agents to the ends of one or more fragmented nucleic acid molecules.

In certain embodiments, the amplified or replicated dumbbell templates can be detected with oligonucleotide probes. The oligonucleotide probes can be labeled oligonucleotide probes. The oligonucleotide probes can be labeled DNA probes. In certain embodiments, the oligonucleotide probe can be attached to one or more of a fluorophore, a chromophore, a radioisotope, an enzyme, or a luminescent compound, or combinations thereof.

Certain hairpin structures have also been used as oligonucleotide probes. Certain DNA probes, also known as molecular beacons, are oligonucleotides designed to contain an internal probe sequence with two ends that are complementary to one another. Under appropriate conditions, the ends hybridize together forming a stem-loop structure. The probe sequence is contained within the loop portion of the molecular beacon and is unrelated to the stem arms. A fluorescent dye is attached to one end on the stem and a non-fluorescent quenching moiety or "quencher" is attached to the other end of the stem. In the stem-loop configuration, the hybridized arms keep the fluorescent dye and quencher in close proximity, resulting in quenching of the fluorescent dye signal by the well-understood process of fluorescence resonance energy transfer (FRET). When the probe sequence within the loop structure finds and hybridizes with its intended target sequence, the stem structure is broken in favor of the longer and more stable probe-target duplex. Probe hybridization results in the separation of the fluorescent dye and quencher (*i.e.,* the close proximity is now lost), for which dye can now fluoresce when exposed to the appropriate excitation source of the detector. Molecular beacons have been used in a number of molecular biology techniques, such as real-time PCR, to discriminate allelic differences.

In certain embodiments, the hairpin structures can be created by using two or more nucleic acid molecules that are then joined to form a single hairpin structure. The two or more nucleic acid molecules can be joined together using ligating reagents to form a hairpin structure. The two or more nucleic acid molecules can also be chemically joined together using a linker to form a hairpin structure. In certain embodiments, the 5'-ends of one or more hairpin structures will be phosphorylated, for example and without limitation, using $T_4$ polynucleotide kinase to facilitate the efficient ligation using ligating agents to the ends of one or more fragmented nucleic acid molecules.

In certain embodiments, functionally important information can reside in the stem region of the hairpin structure. In certain embodiments, functionally important information can reside in the loop region of the hairpin structure. Functionally important information can include, for example and without limitation, the necessary sequences for *in vitro* replication, *in vitro* amplification, unique identification (*i.e.,* barcodes), and detection. In certain embodiments where the functionally important information resides in the loop region of the hairpin structure, the length of the stem region can be as few as four or six base-pairs. In certain embodiments where the functionally important information resides in the stem region of the hairpin structure, the length of the loop region can be as few as one or two bases.

Mate-pair template libraries are prepared by circularizing sheared genomic DNA that has been selected for a given size, such as 2-kb, therefore bringing the ends that were previously distant from one another into close proximity.

The circles are then cut by mechanical or physical means into linear DNA fragments. Those DNA fragments containing the ligated distant ends, called junction fragments, are used to create mate-pair templates. A "junction fragment" is a DNA molecule that contains the distant ends of a larger DNA molecule in combination with a selectable marker and was created by making first a DNA circle, fragmenting the DNA circle, and selecting for fragments containing a selectable marker.

For example and without limitation, a method of creating circles involves partially digesting high molecular-weight genomic DNA with a restriction endonuclease, such as *Mbo* I. Other known 4-, 6-, or 8-base "cutters" or the equivalents may also be used. The DNA fragments at very low concentration and in combination with a small, selectable marker are ligated together to create covalent DNA circles. Thus, a circular DNA molecule is generated with the selectable marker flanked by both of the distant ends of the DNA fragment. A library of junction fragments is created by digesting DNA circles with a different restriction endonuclease, such as *Eco*RI, and then selecting for the marker fragment flanked by those distant ends. The junction fragment libraries are used in genetic and physical mapping experiments as well as in sequencing applications.

In more general terms, several factors should be considered when optimizing the ratio of ligating fragments that favor "intramolecular" ligation events (*i.e.,* DNA circles of the same nucleic acid molecule) over "intermolecular" ligation events (*i.e.,* joining of two or more nucleic acid molecules called concatemers). The ratio is governed by two parameters: the effective local molar concentration ($j$) of one end of a molecule experienced by the other end of the *same* molecule and the molar concentration ($i$) of the ends of all *other* DNA molecules. The parameter $j$ can be determined from the Jacobson-Stockmayer equation:

$$j = 3.55 \text{ x } 10^{-8} \text{ M / kb}^{3/2}$$

where kb is the length of the nucleic acid molecule in kilobase-pairs (kpb). For a given ligation reaction, the percentage of intramolecular events is determined by the ratio of $j / (i+j)$. That is, larger nucleic acid molecules must be further diluted compared with smaller nucleic acid molecules in order to achieve a reasonable efficiency in creating intramolecular circles. For a selectable marker to be incorporated during intramolecular ligation with reasonable probability, its molar concentration should be roughly equivalent to $j$. Even under very dilute ligation conditions, however, the probability of forming intermolecular ligation species will still occur, resulting in a mixture of intramolecular circles and linear concatemers of two or more nucleic acids molecules. There are several technical problems associated with creating large circular nucleic acid molecules including (*a*) generating and handling very large nucleic acid molecules without breaking them into smaller nucleic acid molecules, (*b*) identifying an appropriate selectable marker to enrich for the junction fragments, and (*c*) requiring large amounts of starting nucleic acids material in creating complete, representative nucleic acid libraries. Well established methods exist in the art for handling large nucleic acid molecules, such as pulsed-field gel electrophoresis, and alternative strategies have been used, such as the biotin/avidin or streptavidin system, to improve the selection of junction fragments. The issue regarding the need for large amounts of starting nucleic acids material, however, has not been adequately addressed. Thus, creating nucleic acid circles by the strategy of intramolecular ligation events is rarely applicable when considering the analysis of precious biological samples that appear in limited quantities, such as biopsied samples obtained during surgical procedures or free circulating DNA obtained from whole blood, plasma, or serum.

There are numerous methods of isolating a nucleic acid from a sample. Once isolated, one or more nucleic acid molecules may be broken into smaller fragments by the process of fragmenting, for example and without limitation, in a non-sequence-specific or in a sequence-specific manner. The non-sequence-specific or random fragmentation process is expected to produce an even or substantially even distribution of fragmented nucleic acid molecules along a given genome of interest. For example and without limitation, 1,000,000 fragmented nucleic acid molecules could be mapped to 1,000 locations of equal size (*i.e.,* windows) with each window having a count of 1,000 mapped fragmented nucleic acid molecules. In certain embodiments, the data obtained from the even or substantially even distribution of fragmented nucleic acid molecules along a given genome of interest may show bias in favor of certain data types over another, for example and without limitation, GC content of a given region of the genome under investigation. In certain embodiments, nucleic acid molecules are fragmented enzymatically using DNase I, which fragments double-stranded DNA non-specifically. The products of fragmenting are 5'-phosphorylated di-, tri-, and oligonucleotides of differing sizes. DNase I has optimal activity in buffers containing $Mn^{2+}$, $Mg^{2+}$, and $Ca^{2+}$, but having no other salts in the buffer. Fragmenting using DNase I will typically result in a random digestion of the double-stranded DNA with a predominance of blunt-ended double-stranded DNA fragments when used in the presence of $Mn^{2+}$ based buffers. Even under the use of $Mn^{2+}$ based buffer conditions, fragmented nucleic acid molecules may contain 5'-protuding ends of one or more single-stranded nucleotides of unknown sequence extending beyond the end of the other nucleic acid strand of the fragmented duplex, referred to here as "5'-end overhangs") and 3'-protruding ends of one or more single-stranded nucleotides of unknown sequence extending beyond the end of the other nucleic acid strand of the fragmented duplex, referred to here as "3'-end overhangs"). The range of fragment sizes of the library following DNase I digestion are dependent on several factors, for example and without limitation, (*i*) the amount (in units) of DNase I used in the reaction, (*ii*) the temperature of the reaction, and (*iii*) the time of the reaction.

In certain embodiments, nucleic acid molecules are fragmented in a non-sequence-specific manner using physical or mechanical means. For example and without limitation, nucleic acid molecules can be fragmented using nebulization, which shears double-stranded nucleic acid molecules in smaller fragments. The range of fragment sizes of the library following nebulization are dependent on several factors, for example and without limitation, (*i*) the pressure applied to the nebulizer and *(ii)* the time of the shearing process. The sheared library of fragments contain a variety of end types including blunt-ended, 5'-end overhangs, and 3'-end overhangs. The ends of one or more fragmented nucleic acid molecules using random fragmenting methods can be ligated to adaptors to form dumbbell templates directly using ligating agents or be made capable of ligating dumbbell templates, see below.

Isolated nucleic acid molecules from a sample may also be broken into smaller fragments by a sequence-specific fragmenting process, for example and without limitation, using one or more restriction endonucleases. The sequence-specific fragmentation process is expected to produce an uneven or substantially uneven distribution of fragmented nucleic acid molecules along a given genome of interest. For genome-wide studies, the sequence-specific fragmenting process may not be optimal as some fraction, which may be significant, of genome regions will be expected to have a low frequency of restriction endonuclease cleavage sites. The distribution of cleavage sites is dependent of the type and number of restriction endonucleases used for a given fragmenting process. Regions with low frequencies of cleavage sites will result in an under-representation of genome information. There are several advantages of using the sequence-specific fragmenting approach, for example and without limitation, targeting a subset of the genome of interest, which reduces efforts, costs, and data analysis and the ends of the fragmented nucleic acid molecules will be defined as blunt-ends or as defined 5'-end overhang nucleic acid sequences and defined 3'-end overhang nucleic acids sequences. Protruding ends with defined nucleic acid sequences are referred to as "sticky ends." In certain embodiments, two or more restriction endonucleases may be used to create smaller fragments with each end having a different sticky end sequence. For example and without limitation, the isolated nucleic acid molecules are digested with two restriction endonucleases (*i.e., Eco*RI and *Bam*HI), which will result in three different sticky end types *(i.e.,* both ends containing either the same 5'-overhang sequence of 5'-AATT [*Eco*RI] or 5'-overhang sequence of 5'-GATC *[BamHI]* or both ends containing different sticky ends *(i.e.,* one end having the 5'-overhang sequence of 5'-AATT and the other end having the 5'-overhang sequence of 5'-GATC). One hairpin structure having a complementary sticky end of 5'-AATT can be joined using ligating agents to fragments containing *EcoRI* sticky ends and a different hairpin structure having a complementary sticky end of 5'-GATC can be joined using ligating agents to fragments containing *Bam*HI sticky ends. Dumbbell templates containing different hairpin structures may be enriched using affinity supports that contain complementary sequences for the different hairpin structures, see examples for more detail.

Isolated nucleic acid molecules from a sample may also be created into smaller fragments by a sequence-specific fragmenting process, for example and without limitation, using multiplex PCR. For example and without limitation, two or more PCR primer sets may be designed to specifically amplify two or more target regions comprising nucleic acid molecules. In addition to designing target-specific nucleic acid sequences comprising the primer, additional nucleic acid sequences having functionally important information can include, for example and without limitation, one or more restriction endonuclease cleavage sites and unique identification (*i.e.,* barcodes). In certain embodiments, one or more forward PCR primers may contain one given restriction endonuclease cleavage site and one or more reverse PCR primers may contain one different restriction endonuclease cleavage site. Upon contacting the amplified PCR products with corresponding restriction endonucleases, each of which recognizes and cuts its cleavage site, the ends of the amplified PCR products may contain different sticky ends, which may be used for the attachment of two different hairpin structures in a predictable manner. For example and without limitation, in addition to target-specific nucleic acid sequences, all forward PCR primers contain an *Eco*RI restriction endonuclease cleavage site and all reverse PCR primers contain a *Bam*HI restriction endonuclease cleavage site. Following multiplex PCR using two or more PCR primer sets, restriction endonuclease digestion of the amplified PCR products with *Eco*RI and *Bam*HI will result in the forward primer ends having a 5'-overhang sequence of 5'-AATT and the reverse primer ends having a 5'-overhang sequence of 5'-GATC. One hairpin structure having a complementary sticky end of 5'-AATT can be joined using ligating agents to only the forward primer end and a different hairpin structure having a complementary sticky end of 5'-GATC can be joined using ligating agents to only the reverse primer end. In some embodiments, isolated nucleic acid molecules from a sample may not require any fragmenting process as these isolated nucleic acid molecules may be sufficiently fragmented for creating dumbbell templates. For example and without limitation, isolated nucleic acid molecules from serum or plasma from whole blood obtained from pregnant females or cancer patients are sufficiently fragmented *in vivo* that additional fragmenting may not be necessary for creating dumbbell templates. In certain embodiments, samples can be obtained from cancer patients. In certain embodiments, samples can be obtained from pregnant individuals. In certain embodiments, samples can be obtained from pathology specimens. In certain embodiments, samples can be obtained from formalin-fixed paraffin-embedded (FFPE) specimens. In certain embodiments, samples can be obtained from environmental samples. In certain embodiments, the nucleic acid molecules can be of lengths ranging from greater than 2.5 kbp to 100 kbp.

The isolated *in vivo* fragmented library of nucleic acid molecules contain a variety of end types including blunt-ended, 5'-end overhangs, and 3'-end overhangs. The ends of a fragmented nucleic acid molecule can be ligated to adaptors to form ligated dumbbell templates directly using ligating agents or be first processed such that the ends can form ligating dumbbell templates, see below.

The percentage of fragmented nucleic acid molecules containing blunt-ends can be increased with the use of polishing methods, for example and without limitation, by using polymerases that exhibit 3'-exonuclease activity. For example and without limitation, such polymerases may include $T_4$ DNA polymerase, Klenow DNA polymerase, or *Pfu* DNA polymerase. The 3'-exonuclease activity of these DNA polymerase functions by removing the one or more single-stranded nucleotides of unknown or known sequence from 3'-end overhangs to create a blunt-ended fragmented nucleic acid molecules. The 5'-end overhangs are made blunt-ended by the enzymatic incorporation of complementary nucleotides to the recessed 3'-end strand to also create blunt-ended fragmented nucleic acid molecules. In certain embodiments, the 5'-ends of one or more fragmented nucleic acid molecules can be phosphorylated, for example and without limitation, using $T_4$ polynucleotide kinase to facilitate the efficient creation of dumbbell templates using ligating agents to the ends of one or more hairpin structures.

In certain embodiments following the blunt ending and phosphorylating of fragmented nucleic acid molecules, double-stranded oligonucleotide adapters can be designed to introduce functionally important information, for example and without limitation, replication, amplification, and/or unique identification *(i.e.,* barcodes) sequences as well as providing any given sticky end sequence. The latter sequence can be useful to facilitate efficient creation of dumbbell templates using ligating agents with 5'-phosphorylated hairpin structures having complementary sticky end sequences. In certain embodiments, a transposase and transposon complex can be used in fragmenting one or more nucleic acid molecules and simultaneously inserting functionally important information, for example and without limitation, replication, amplification, and/or unique identification *(i.e.,* barcodes) sequences as well as providing any given restriction endonuclease cleavage site capable of creating sticky end sequences at the point of insertion. In certain embodiments, the ends of a fragmented nucleic acid molecule can be modified by other means, for example and without limitation, by the addition of a 2'-deoxyadenosine (dA) nucleotide to the 3'-end of the blunt-ended fragmented nucleic acid molecule. For example and without limitation, DNA polymerases that lack a 3'-exonuclease activity, such as Klenow 3'-exo minus DNA polymerase and *Taq* DNA polymerase can add 2'-deoxyadenosine triphosphates to the 3'-ends of blunt-ended fragmented nucleic acid molecules to yield a 3'-end overhang with one 2'-deoxyadenosine monophosphate nucleotide. The dA-tailing method also facilitates efficient dumbbell template construction using ligating agents with 5'-phosphorylated hairpin structures having complementary 3'-end overhang of one 2'-thymidine monophosphate nucleotide. In certain embodiments, the blunt-ended fragmented nucleic acid molecules can also be directly used to create dumbbell templates using ligating agents to join 5'-phosphorylated hairpin structures having corresponding blunt-ends.

Unlike current methods that create nucleic acid circles by the strategy of intramolecular ligation events, which is rarely applicable when considering the analysis of precious biological samples in limited quantities, the efficiency of creating nucleic acid circles is greatly improved by the methods of the present invention. For example and without limitation, creating nucleic acid circles by the strategy of intramolecular ligation events requires tens of micrograms of starting material, yet yields an approximate efficiency of one (1) percent or less in creating the desired nucleic acid circles. The problems associated with the intramolecular ligation strategy is further compounded as the method is dependent on the size of the nucleic acid molecule and is inversely proportional to the efficiency of ligation. That is, nucleic acid molecules of bigger size create fewer circles by the intramolecular ligation approach because the reaction conditions dictate increasingly dilute concentrations that are proportional to the length of the nucleic acid molecule. On the other hand, the methods described in the present invention to create dumbbell templates are highly efficient as the methods do not rely on the intramolecular ligation approach. On the contrary, the creation of dumbbell templates is performed by intermolecular events, where the ligation efficiency of joining nucleic acid molecules to hairpin structures can be made very efficient. The ligation reaction can proceed to completion or substantially near completion as the concentration of the hairpin structures can be sufficiently high *(i.e.,* 100-fold) above the concentration of the nucleic acid molecule. The ligation reaction is also independent or substantially independent of the size of one or more nucleic acid molecules, as dumbbell templates of a size of 1,000 bp can be created as efficiently as dumbbell templates of a size of 5,000 bp or a size of 10,000 bp or even a size of 100,000 bp, or even larger than 100,000 bp. In certain embodiments of the invention, efficient, dual-hairpin dumbbell templates in size increments of 5.0, 7.5, and 10.0 kb may be constructed from genomic DNA. In certain embodiments, dumbbell templates may then be replicated or amplified using a rolling circle mechanism in a homogeneous reaction solution. In certain embodiments, dumbbell templates may also be replicated or amplified using a rolling circle mechanism in a heterogeneous reaction solution using one or more solid-phase bound primers by introducing the dumbbell templates onto the substrate in limiting dilution such that one or more replicated dumbbell templates or amplified dumbbell templates are spatially and spectrally resolvable for detecting a nucleic acid molecule.

In certain embodiments, the dumbbell template is a plus strand nucleic acid molecule containing one or more hairpin structures. In certain embodiments, a dumbbell template can also be formed whereby each end of a single-stranded nucleic acid molecule is ligated to hairpin structure, whereby one hairpin structure can act as a primer to extend and copy the single-stranded nucleic acid molecule to the other end of the hairpin structure. Following a ligating step, the dumbbell template is formed. In certain embodiments of the invention, the linear double-stranded region may be melted, for example and without limitation, by heat, chemical or enzymatic means, and the dumbbell template can be transformed into a fully-open, single-stranded circle. In certain embodiments, the dumbbell templates may be created using two different hairpin structures having different nucleic acid sequences containing unique restriction endonuclease cleavage sites. These circular templates may be replicated using the rolling circle mechanism to create multiple copies of the target sequence. Following the RCR step, the linear concatemers may be digested with an appropriate restriction endonuclease to produce monomer units of the target sequence, the ends of which were then ligated together to create multiple copies of circular target sequences. In certain other embodiments of the invention, the dumbbell templates may be used in the transcription of RNA molecules of the gene of interest. Dumbbell templates containing one or more RNA promoter sequences are generated and these closed single-stranded nucleic acid circles are used as templates for *in vitro* transcription of RNA molecules of the gene of interest.

In certain embodiments, exonucleases can be used to remove undesired nucleic acid molecules that did not successfully ligate to form dumbbell templates. These undesired nucleic acid molecules may have one or more 5'-ends or 3'ends that may be in the form of a blunt-ended, 5'-protuding ends, and/or 3'-protruding ends, or may exist in single-stranded form. These undesired nucleic acid molecules include, but are not limited to, unfragmented and fragmented nucleic acid molecules, oligonucleotides that may not have formed into a hairpin structure, and unligated hairpin structures. Exonuclease III (also called Exo III) catalyzes the stepwise removal of mononucleotides from 3'-hydroxyl termini of double-stranded DNA. A limited number of nucleotides are removed during each binding event, resulting in coordinated progressive deletions within the population of DNA molecules. The preferred substrates of Exo III are nucleic acid molecules containing blunt-ends or 5'-protuding ends, although the enzyme also acts at nicks in double-stranded DNA to produce single-strand gaps. Exo III is not active on single-stranded DNA, and thus 3'-protruding ends are resistant to cleavage. The degree of resistance depends on the length of the extension, with extensions four bases or longer being essentially resistant to cleavage. This property can be exploited to produce unidirectional deletions from a linear molecule with one resistant (3'-protruding ends) and one susceptible (blunt-ends or 5'-protruding ends) terminus. Exonuclease III activity depends partially on helical structure and displays sequence dependence (C>A=T>G). Temperature, salt concentration and the ratio of enzyme to DNA greatly affect enzyme activity, requiring reaction conditions to be tailored to specific applications. Exonuclease VII (also called Exo VII) cleaves single-stranded DNA from both 5'→3' and 3'→5' direction. This enzyme is not active on linear or circular double-stranded DNA. It is useful for removal of single-stranded oligonucleotide primers and hairpins from a completed PCR reaction and post-ligation reactions when creating dumbbell templates. Digestion of single-stranded DNA by Exonuclease VII is metal-independent. Exo III and Exo VII can be used in combination to remove undesired nucleic acid molecules that did not successfully ligate to form dumbbell templates.

The substrate can be comprised of any material, for example and without limitation, a solid material, a semi-solid material *(i.e., [i]* a composite of a solid support and a gel or matrix material or [i*i]* linear or cross-linked polyacrylamide, cellulose, cross-linked agarose, and polyethylene glycol), or fluid or liquid material. The substrate can also be comprised of any material that has any dimensions and shape, for example and without limitation, square, trapezoidal, spherical, spheroidal, tubular, pellet-shaped, rod-shaped, or octahedral. The substrate should contain properties that are compatible with the present invention *(i.e.,* exhibit minimal interference with replication, amplification, or detection processes). In certain embodiments, the substrate is nonporous. In certain embodiments, the substrate is porous. In certain embodiments, the substrate can be comprised of a hydrophilic porous matrix, such as a hydrogel. In certain embodiments, the solid material comprises, for example and without limitation, a glass material *(i.e.,* borosilicate, controlled pore glass, fused silica, or germanium-doped silica), silicon, zirconia, titanium dioxide, a polymeric material *(i.e.,* polystyrene, cross-linked polystyrene, polyacrylate, polymethylacrylate, polydimethylsiloxane, polyethylene, polyfluoroethylene, polyethyleneoxy, polypropylene, polyacrylamide, polyamide such as nylon, dextran, cross-linked dextran, latex, cyclic olefin polymer, cyclic olefin copolymer, as well as other co-polymers and grafts thereof), or a metallic material. Solid substrates can consist, for example and without limitation, of one or more membranes, planar surfaces, substantially planar surfaces, non-planar surfaces, microtiter plates, spherical beads, non-spherical beads, fiber-optics, fiber-optics containing spherical beads, fiber-optics containing non-spherical beads, semi-conductor devices, semi-conductor devices containing spherical beads, semi-conductor devices containing non-spherical beads, slides with one or more wells containing spherical beads, slides with one or more wells containing non-spherical beads, filters, test strips, slides, cover slips, or test tubes. In certain embodiments, the semi-solid material comprises, for example and without limitation, linear or cross-linked polyacrylamide, cellulose, cross-linked agarose, and polyethylene glycol.

One or more primers can be attached to a substrate by any suitable means. In certain embodiments, the attachment of one or more primers to the substrate, for example and without limitation, is mediated by covalent bonding, by hydrogen bonding *(i.e.,* whereby the primer is hybridized with another complementary oligonucleotide covalently attached to the substrate and still serves a replication competent or amplification competent function), Van Der Waal forces, physical adsorption, hydrophobic interactions, ionic interactions or affinity interactions *(i.e.,* binding pairs such as biotin/streptavidin or antigen/antibody). In certain embodiments, one member of the binding pair is attached to the substrate and the other member of the binding pair is attached to one or more primers. The attachment of one or more primers to the substrate occurs through the interaction of the two members of the binding pair.

The order by which one or more primers are attached to the substrate can be of any arrangement, broadly defined as the "primer array," for example and without limitation, in random arrays, by random assortment in patterned array, or by knowns patterned in ordered arrays. Primer arrays that replicate dumbbell templates by a rolling circle mechanism are broadly defined as "replicated dumbbell template arrays." Primer arrays that amplify dumbbell templates by a rolling circle mechanism are broadly defined as "amplified dumbbell template arrays." By design, patterned arrays and ordered arrays are expected to provide replicated dumbbell template arrays or amplified dumbbell template arrays that are spatially and spectrally resolvable for detecting a nucleic acid molecule. In certain embodiments of a random array, one or more primers can be covalently bonded to the substrate to form a high-density lawn of immobilized primers on a planar or substantially planar surface. The one or more primers may be attached by any means, for example and without limitation, by methods involving dropping, spraying, plating or spreading a solution, emulsion, aerosol, vapor, or dry preparation. By introducing dumbbell templates onto the substrate in limiting dilution fashion, one or more primers will contact the dumbbell template, enabling the rolling circle mechanism in the presence of polymerase to produce one or more replicated dumbbell templates *(i.e.,* replicated dumbbell template array) or amplified dumbbell templates *(i.e.,* amplified dumbbell template array) that are spatially and spectrally resolvable for detecting a nucleic acid molecule. In certain embodiments of a random assortment in patterned arrays, one or more primers can be covalently bonded to the substrate to form high-density, immobilized primers on one of more spherical or non-spherical beads. By introducing dumbbell templates onto the substrate in limiting dilution using an oil in water emulsion system, one or more primers will contact the dumbbell template, enabling the rolling circle mechanism to produce one or more replicated dumbbell templates or amplified dumbbell templates. In certain embodiments, replicated dumbbell templated beads or amplified dumbbell templated beads can be enriched to remove those beads that failed to replicate or amplify dumbbell templates based on Poisson statistics of distributing single molecules. Replicated dumbbell templated beads or amplified dumbbell templated beads, with or without enrichment, can then be distributed randomly in an ordered pattern on planar or substantially planar slide substrate, fiber-optic substrate, or, semi-conductor device substrates containing wells, depressions, or other containers, vessels, features, or locations. In other certain embodiments of a random assortment in patterned arrays, one or more prefabricated hydrophilic features *(i.e.,* spots) on the surface can be surrounded by hydrophobic surfaces for the covalent bonding of one or more primers to the substrate. For example and without limitation, patterned arrays can be created photolithographically etched, surface modified silicon substrates with grid-patterned arrays of ~300 nanometer spots. By introducing the dumbbell templates onto a patterned substrate in limiting dilution fashion, the primer will contact the dumbbell template, enabling the rolling circle mechanism to produce one or more replicated dumbbell templates or amplified dumbbell templates. In certain embodiments, the prefabricated hydrophilic spots can be made small even to accommodate only one replicated dumbbell template or amplified dumbbell template. As distributing single molecules based on Poisson statistics results in a considerable fraction of no template spots, following the rolling circle procedure, additional rounds of distributing, contacting, and rolling circle may be employed to increase the density of replicated dumbbell templates or amplified dumbbell templates on the substrate. In certain embodiments of "knowns" patterned in ordered arrays, one or more known primers can be printed *(i.e.,* spotted arrays) or made *in situ* at addressable locations on the substrate. By introducing the dumbbell templates onto a patterned substrate in limiting dilution fashion, one or more primers will contact the dumbbell template, enabling the rolling circle mechanism to produce one or more replicated dumbbell templates or amplified dumbbell templates.

The polymerase chain reaction ("PCR") is used to specifically amplify a small amount of nucleic acid molecules, generating thousands to millions of copies of the target sequence of interest. Generally speaking, PCR involves repeated heating to denature or melt the duplex strands, cooling to hybridize the primers, and then heating again (usually at the optimal temperature for DNA polymerase, but below the denaturation temperature) to amplify the template sequences *in vitro (i.e.,* outside of an organism). DNA polymerase copies or synthesizes the complementary strand from a single-stranded template. For this enzymatic reaction to occur, a partially double-stranded section of DNA is required. Typically, a primer hybridizes to a complementary region of a single-stranded template. DNA polymerase synthesizes the nascent strand in a 5'-to-3' direction to create double-stranded DNA. Multiplex PCR allows for the simultaneous amplification of multiple target regions and has been used to detect coding exon deletion(s) in X-linked disorders (these exons are gene sequences that are transcribed into messenger RNA (mRNA) and translated into one or more proteins); such X-linked disorders include Duchenne muscular dystrophy and Lesch-Nyhan syndrome. In the alternative, one can use PCR to amplify an entire pool of nucleic acids present in the starting mixture, resulting in the amplification, but not targeted enrichment, of any given subset of nucleic acids. This is accomplished by ligating common sequences or adapters to the end of the fragments, and amplifying the fragments by denaturing the fragments, hybridizing common primers whose sequences are complementary to the common adapters, and copying the DNA fragment. This type of PCR is called "universal PCR."

Bacteriophages (or phages), such as ΦX174, M13, lambda, and some viruses can replicate their respective genomes by a "rolling circle" mechanism. An entire genome is reproduced by copying from a circular template. Unlike PCR, the rolling circle mechanism can be performed isothermally (i.e., that is without the need for heating or cooling cycles).

The rolling circle approach has been used as an in vitro method for replicating (i.e., using one or more primers that copy only original dumbbell templates) or amplifying (i.e., using two or more primers that copy both original dumbbell templates as well as copies of dumbbell templates) nucleic acid molecules of interest. For example, circular synthetic oligonucleotide templates, ranging from 34-to-52 bases in size, have been replicated using a rolling circle mechanism using E. coli Pol I DNA polymerase and a single oligonucleotide primer. The rolling circle mechanism using similar size circles, range 26-to-74, with several polymerases, including E. coli Pol I, Klenow DNA polymerase, and $T_4$ DNA polymerases.

In certain embodiments, DNA circles can be created as "padlock probes." A major disadvantage with the padlock approach is the size limitation of creating circular nucleic acid molecules, for example and without limitation 46-nucleotide circles used to target the CFTR G542X gene locus. These padlock circles can be useful using DNA polymerases, such as φ29 DNA polymerase, Bst DNA polymerase, and Vent(exo-) DNA polymerase to create hundreds of target copies in just a matter of minutes. In certain embodiments, padlock circles can use two primers in the rolling circle amplification mechanism, which enabled copying of not only the template circle (i.e., the minus strand), but also enabled copying of the newly synthesized plus strand(s). The RCA method using two different 46-nucleotide padlock circles can be used for genotyping applications, for example and without limitation, the detection of a wild-type sequence and a mutant sequence for the CFTR G542X gene locus. Another disadvantage of the RCA padlock method for genotyping is the requirement of individual allele-discriminating primers for each mutational locus being assayed.

Rolling circle amplification has been used in Sanger sequencing application using random hexamers (i.e., more than two primers) and φ29 DNA polymerase for solution-based template preparation using traditional cloning sources, such as plasmids and phage as DNA circles, ranging in size from 5-to-7-kb in size. A disadvantage of using traditional cloning approaches in creating DNA circles is the requirement to propagate such DNA circles via an appropriate cellular host. Dumbbell templates of the present invention overcome this limitation. Rolling circle replication using chimeric DNA templates has been used in a sequencing-by-ligation method. The template preparation method used a complicated series of directional adapter ligations and Type IIs restriction enzyme digestions to create small DNA circles approximately 300 bp in size by an intramolecular ligation approach, which are replicated in solution using a single primer and φ29 DNA polymerase to create "DNA nanoballs." These nanoballs are then absorbed onto a patterned substrate to perform their sequencing-by-ligation method. A major limitation of the nanoball method is that the amount of genomic DNA sequence available in the chimeric template circle is small (i.e., 76-bp is actual target sequence and the remaining 222-bp is adapter sequences) and the requirement of intramolecular ligation. The present invention overcomes the limitations of complex methods to construct small circular templates by intramolecular ligation approaches by providing a simpler workflow using dumbbell templates that can be replicated or amplified in a rolling circle mechanism.

Polymerases and reverse transcriptases that are useful in a rolling circle mechanism generally exhibit the property of strand-displacement, which is the ability to displace a "downstream" nucleic acid strand encountered by the enzyme during nucleic acid synthesis. These strand-displacing enzymes also lack 5'-exonuclease activity. Any strand-displacing polymerase or reverse transcriptase can be used in rolling circle replication or rolling circle amplification, for example and without limitation, φ29 DNA polymerase, E. coli Pol I, Klenow DNA polymerase, Bst DNA polymerase (large fragment), Bsm DNA polymerase (large fragment), Bsu DNA polymerase (large fragment), Vent(exo-) DNA polymerase, $T_7$ (exo-) DNA polymerase ($T_7$ Sequenase), or TopoTaq (a chimeric protein of Taq DNA polymerase and topoisomerase V), as well as mutant versions of these DNA polymerases thereof, $T_7$ RNA polymerase, $T_3$ RNA polymerase, or SP6 RNA polymerase as well as mutant versions of these RNA polymerases thereof, or avian myeloblastosis virus reverse transcriptase or Moloney murine leukemia virus reverse transcriptase, as well as mutant versions of these reverse transcriptases, such as ThermoScript reverse transcriptase, SuperScript reverse transcriptase or PrimeScript reverse transcriptase. In addition to strand-displacing polymerases and reverse transcriptases, accessory proteins can further enhance the displacement of a downstream nucleic acid strand during nucleic acid synthesis by increasing the robustness, fidelity, and/or processivity of the rolling circle mechanism. Strand-displacing accessory proteins can be of any type and include, for example and without limitation, helicases, single-stranded binding proteins, topoisomerases, reverse gyrases, and other proteins that stimulate accessory proteins, for example and without limitation, E. coli MutL protein or thioredoxin. DNA helicases are useful in vivo to separate or unwind two complementary or substantially complementary DNA strands during DNA replication. Helicases can unwind nucleic acid molecules in both a 5'-to-3' direction, for example and with limitation, bacteriophage $T_7$ gene 4 helicase, DnaB helicase and Rho helicase and a 3'-to-5' direction, for example and with limitation, E. coli UvrD helicase, PcrA, Rep, and NS3 RNA helicase of hepatitis C virus. Helicase may be obtained from any source and include, for example and without limitation, E. coli helicases (i.e., I, II [UvrD], III, and IV, Rep, DnaB, PriA and PcrA), bacteriophage $T_4$ gp41, bacteriophage $T_7$ gene 4 helicase, SV40 Large T antigen, Rho helicase, yeast RAD helicase, thermostable UvrD helicases from *T. tengcongensis,* and NS3 RNA helicase of hepatitis C virus, as well as mutant versions of these and other helicases. Single-stranded binding protein binds single-stranded DNA with greater affinity that double-stranded DNA. These proteins bind cooperatively, favoring the invasion of single-stranded regions and therefore destabilizing duplex structures. For example and without limitation, single-stranded binding protein can exhibit helix-destabilizing activity by removing secondary structure and can displace hybridized nucleic acid molecules. Single-stranded binding proteins may be obtained from any source and include, for example and without limitation, bacteriophage $T_4$ gene 32 protein, RB 49 gene 32 protein, *E. coli* single-stranded binding protein, $\varphi$29 single-stranded binding protein or bacteriophage $T_7$ gene 2.5, as well as mutant versions of these and other single-stranded binding proteins, such as bacteriophage $T_7$ gene 2.5 F232L.

The dumbbell templates can be subject to a rolling circle replication using highly processive, strand-displacing polymerases, such as phi29 polymerase. The rolling circle replication can be performed in two steps. First, size-selected dumbbell templates are allowed to hybridize with dumbbell complementary primers under appropriate "hybridization conditions," which include temperature, factors such as salts, buffer and pH, detergents, and organic solvents. Blocking agents such as Bovine Serum Albumin (BSA) or Denhardt's reagent may be used as part of the hybridization conditions. Second, an appropriate polymerase or replisome and nucleotide mix are provided to the first reaction mixture to produce amplified or replicated dumbbell templates. The hybridization and amplification or replication conditions are optimized based on several factors, including but not limited, to the length and sequence composition of the stem region of the dumbbell templates, the hybridization conditions, the specific polymerase or replisome used herein, and the reaction temperature. In certain embodiments, the reaction temperature can be about 10°C to 35°C. In other embodiments, the reaction temperature can be about 15°C to 30°C. In other embodiments, the reaction temperature can be about 20°C to 25°C. In certain embodiments, the temperature is increased in select time intervals. For example, without limitations, the reaction is maintained for five minutes at 10°C, then five minutes at 15°C, five minutes at 20°C, then five minutes at 25°C, and five minutes at 30°C.

Replication complexes called "replisomes" may be formed *in vitro* to enhance the rolling circle method by making more copies of replicated dumbbell templates or amplified dumbbell templates and/or replicating or amplifying larger dumbbell templates *(i.e.,* >1 kb, >5 kb, >10 kb, and >50 kb in size). Strand-displacing accessory proteins comprising helicases, single-stranded binding proteins, topoisomerases, and reverse gyrases can be configured with strand-displacing polymerases and reverse transcriptases in any combination to create a replication competent or amplification competent replisome complexes for the rolling circle method of dumbbell templates. In certain embodiments, the combination of $\varphi$29 DNA polymerase and $\varphi$29 single-stranded binding protein under appropriate reaction conditions can enhance the elongation of the rolling circle mechanism by several fold. In certain embodiments, the combination of polymerases or reverse transcriptases that rely on coordinated activities of helicases and single-stranded binding proteins can be used in rolling circle methods to replicate dumbbell templates or amplify dumbbell templates of 10 kb or larger. For example and without limitation, 10 kb plasmids can be amplified using the coordinated activities of $T_7$ Sequenase, $T_7$ helicase, and $T_7$ single-stranded binding protein by forming a replisome complex.

Certain embodiments of the invention include the efficient creation of 10 kb size, dual-hairpin dumbbells with a highly processive, solid-phase RCR system. In certain aspects, uniquely selectable, method-specific, dual-hairpin dumbbell templates are created in a size independent and tightly distributed manner *(i.e.,* 10 $\pm$ 1 kb) allowing for informative downstream bioinformatics processing for de novo assemblies. The creation of dumbbell templates eliminates the need for large quantities of starting genomic DNA as these constructs are made efficiently *(i.e.,* intermolecular vs. intramolecular ligations) with a simple workflow. This is an important consideration when using clinical samples, which are usually obtained in minute quantities. Embodiments of the invention also provide for the development and optimization of solid-phase RCR that relaxes current size constraints imposed by available polymerases representing a technological break-through in NGS technologies. These innovative large template high-density arrays will enable true de novo assembly of complex, novel and disease genomes for research, clinical, and diagnostic applications, and also permit more comprehensive systems biology studies to investigate genome-wide DNA-DNA, DNA-RNA, and DNA-protein interactions.

Replicated dumbbell templates and amplified dumbbell templates attached to a substrate *(i.e.,* replicated dumbbell template array or amplified dumbbell template array) can be useful for many different purposes including, for example and without limitation, all aspects of nucleic acid sequencing, *(i.e.,* whole genome *de novo* sequencing; whole genome resequencing for sequence variant detection, structural variant detection, determining the phase of molecular haplotypes and/or molecular counting for aneuploidy detection; targeted sequencing of gene panels, whole exome, or chromosomal regions for sequence variant detection, structural variant detection, determining the phase of molecular haplotypes and/or molecular counting for aneuploidy detection; as well as other targeted sequencing methods such as RNA-seq, Chip-seq, Methyl-seq, etc; all types of sequencing activities are defined here broadly as "sequencing"). Replicated dumbbell template arrays and amplified dumbbell template arrays can also be useful for creating nucleic acid molecule arrays to study nucleic acid - nucleic acid binding interactions, nucleic acid - protein binding interactions *(i.e.,* fluorescent ligand interaction profiling that quantitatively measures protein-DNA affinity), and nucleic acid molecule expression arrays (*i.e.*, to transcribe 2'-deoxyribonucleic acid molecules into ribonucleic acid molecules, defined here as a "ribonucleic template array") to study nucleic acid structure/function relationships. In certain embodiments, structure/function arrays can be useful for testing the effects of small molecule inhibitors or activators or nucleic acid therapeutics, for example and without limitation, therapeutic antisense RNA, ribozymes, aptamers, and small interfering RNAs, that can perturb one or more structure/function relationships of the ribonucleic template array, as well as detect nucleic acid - nucleic acid binding interactions and nucleic acid - protein binding interactions. In certain embodiments of the invention, ribonucleic template arrays can be further translated into their corresponding amino acid sequence, defined here as "protein arrays," that can be useful, for example and without limitation, to study protein - nucleic acid binding interactions and protein - protein binding interactions, for screening of ligands (particularly orphan ligands) specific for one or more associated protein receptors, drug screening for small molecule inhibitors or activators or nucleic acid therapeutics, for example and without limitation, therapeutic antisense RNA, ribozymes, aptamers, and small interfering RNAs that can perturb one or more structure/function relationships of the protein array.

In certain embodiments, replicated dumbbell template arrays and amplified dumbbell template arrays can be useful for more than just one purpose by providing additional information beyond single purpose uses, for example and without limitation, whole genome de novo sequencing followed by detection of nucleic acid - protein binding interactions for the identification of sequence-specific nucleic acid - protein motifs. An advantage of the present inventions over DNA arrays that rely on solid-phase methods to amplify fragments of 700 bp or less are the replication or amplification of large nucleic acid molecules of at least > 5 kb, or preferably 10 kb, and most preferably 50 kb. Replicated dumbbell template arrays and amplified dumbbell template arrays of increasing template size can capable further information, such a cooperative long-range interactions of two or more nucleic acid - protein binding interaction events along a nucleic acid molecule.

The present inventions will be described more fully hereinafter with reference to the accompanying drawings in which embodiments of the invention are shown. These inventions may, however, be embodied in many different forms and should not be construed as limited to the exemplary embodiments set forth herein; rather, these embodiments are provided so that this disclosure will be thorough and complete, and will fully convey the scope of the inventions to those skilled in the art.

In certain embodiments of the invention, efficient production of circular DNA molecules *via* dumbbell templates has been combined with solid-phase rolling circle replication to create clonally-replicated, large-insert (10-kb in size) replicated dumbbell templates, compatible with the many different purposes described above. In certain embodiments of the invention, efficient production of circular DNA molecules *via* dumbbell templates has been combined with solid-phase rolling circle amplification to create clonally-amplified, large-insert (10-kb in size) amplified dumbbell templates, compatible with the many different purposes described above. Dumbbell templates are created efficiently and are independent of fragmented nucleic acid molecule size, overcoming a significant limitation in current next-generation sequencing methods. The rolling circle replication method or rolling circle amplification method using dumbbell templates overcomes a major limitation of fragmented nucleic acid molecule size, observed in other solid-phase amplification methods such as emulsion PCR and solid-phase amplification.

One embodiment of the invention is a method of replication of at least one dumbbell template, the method containing the steps of fragmenting at least one nucleic acid molecule to form at least one fragmented double-stranded nucleic acid molecule with a minimum length of greater than 5 kb; joining using ligating agents hairpin structures to each end of the at least one fragmented nucleic acid molecule to form at least one dumbbell template; contacting the at least one dumbbell template with at least one substantially complementary primer, wherein the at least one substantially complementary primer is attached to at least one substrate; and performing rolling circle replication on the at least one dumbbell template contacted with the at least one substantially complementary primer to form at least one replicated dumbbell template.

Another embodiment of the invention is a method of amplification of at least one dumbbell template, the method containing the steps of fragmenting at least one nucleic acid molecule to form at least one fragmented double-stranded nucleic acid molecule with a minimum length of greater than 5 kb; joining using ligating agents hairpin structures to each end of the at least one fragmented nucleic acid molecule to form at least one dumbbell template; contacting the at least one dumbbell template with at least two substantially complementary primer, wherein the at least one substantially complementary primer is attached to at least one substrate; and performing rolling circle amplification on the at least one dumbbell template contacted with the at least one substantially complementary primer to form at least one amplified dumbbell template.

Another embodiment of the invention is a method of detecting at least one replicated dumbbell template, the method containing the steps of fragmenting at least one nucleic acid molecule to form at least one fragmented double-stranded nucleic acid molecule with a minimum length of greater than 5 kb; joining using ligating agents hairpin structures to each end of the at least one fragmented nucleic acid molecule to form at least one dumbbell template; contacting the at least one dumbbell template with at least one substantially complementary primer, wherein the at least one substantially complementary primer is attached to at least one substrate; performing rolling circle replication on the at least one dumbbell template contacted with the at least one substantially complementary primer to form at least one replicated dumbbell template; and detecting the at least one replicated dumbbell template. In another embodiment, the step of detecting the at least one replicated dumbbell template consists of sequencing the at least one replicated dumbbell template.

Another embodiment of the invention is a method of detecting at least one amplified dumbbell template, the method containing the steps of fragmenting at least one nucleic acid molecule to form at least one fragmented double-stranded nucleic acid molecule with a minimum length of greater than 5 kb; joining using ligating agents hairpin structures to each end of the at least one fragmented nucleic acid molecule to form at least one dumbbell template; contacting the at least one dumbbell template with at least two substantially complementary primer, wherein the at least one substantially complementary primer is attached to at least one substrate; performing rolling circle amplification on the at least one dumbbell template contacted with the at least one substantially complementary primer to form at least one amplified dumbbell template; and detecting the at least one amplified dumbbell template. In another embodiment, the step of detecting the at least one amplified dumbbell template consists of sequencing the at least one amplified dumbbell template.

Another embodiment of the invention is a method of replication of at least one dumbbell template, the method containing the steps of isolating at least one nucleic acid molecule from a sample; fragmenting at least one nucleic acid molecule to form at least one fragmented double-stranded nucleic acid molecule with a minimum length of greater than 5 kb; joining using ligating agents hairpin structures to each end of the at least one fragmented nucleic acid molecule to form at least one dumbbell template; contacting the at least one dumbbell template with at least one substantially complementary primer, wherein the at least one substantially complementary primer is attached to at least one substrate; and performing rolling circle replication on the at least one dumbbell template contacted with the at least one substantially complementary primer to form at least one replicated dumbbell template.

Another embodiment of the invention is a method of amplification of at least one dumbbell template, the method containing the steps of isolating at least one nucleic acid molecule from a sample; fragmenting at least one nucleic acid molecule to form at least one fragmented double-stranded nucleic acid molecule with a minimum length of greater than 5 kb; joining using ligating agents hairpin structures to each end of the at least one fragmented nucleic acid molecule to form at least one dumbbell template; contacting the at least one dumbbell template with at least two substantially complementary primer, wherein the at least one substantially complementary primer is attached to at least one substrate; and performing rolling circle amplification on the at least one dumbbell template contacted with the at least one substantially complementary primer to form at least one amplified dumbbell template.

Another embodiment of the invention is a method of replication of at least one double-stranded nucleic acid molecule with a minimum length of greater than 5 kb, the method containing the steps of isolating at least one nucleic acid molecule from a sample; joining using ligating agents hairpin structures to each end of the at least one nucleic acid molecule to form at least one dumbbell template; contacting the at least one dumbbell template with at least one substantially complementary primer, wherein the at least one substantially complementary primer is attached to at least one substrate; and performing rolling circle replication on the at least one dumbbell template contacted with the at least one substantially complementary primer to form at least one replicated dumbbell template.

Another embodiment of the invention is a method of amplified of at least one double-stranded nucleic acid molecule with a minimum length of greater than 5 kb, the method containing the steps of isolating at least one nucleic acid molecule from a sample; joining using ligating agents hairpin structures to each end of the at least one nucleic acid molecule to form at least one dumbbell template; contacting the at least one dumbbell template with at least two substantially complementary primer, wherein the at least one substantially complementary primer is attached to at least one substrate; and performing rolling circle amplification on the at least one dumbbell template contacted with the at least one substantially complementary primer to form at least one amplified dumbbell template.

While the embodiments have been described herein with emphasis on the embodiments, it should be understood that within the scope of the appended claims, the embodiments might be practiced other than as specifically described herein. Although the invention has been shown in only a few of its forms, it should be apparent to those skilled in the art that it is not so limited but susceptible to various changes without departing from the scope of the invention.

Those skilled in the art will recognize that many changes and modifications may be made to the method of practicing the invention. In the drawings and specification, there have been disclosed embodiments of the invention and, although specific terms are employed, they are used in a generic and descriptive sense only and not for the purpose of limitation, the scope of the invention being set forth in the following claims. The invention has been described in considerable detail with specific reference to these illustrated embodiments. It will be apparent, however, that various modifications and changes can be made. Furthermore, language referring to order, such as first and second, should be understood in an exemplary sense and not in a limiting sense. For example, those skilled in the art may recognize that certain steps can be combined into a single step.

**Examples**

The following examples further illustrate the compositions and methods.

Example 1

The size independence of dumbbell templates containing two different hairpin structures was demonstrated. A sample DNA, the pUC18 vector was amplified with a set of primers (*i.e.,* forward: 5'-GGA TCC GAA TTC GCT GAA GCC AGT TAC CTT CG and reverse: 5'-GGA TCC GAA TTC AGC CCT CCC GTA TCG TAG TT) to yield a 425 base-pair product. The 5'-ends of each primer contained both *Bam*HI and *Eco*RI restriction enzyme sites. The PCR product then was digested with *Eco*RI to render 5'-AATT overhangs and purified with a QIAquick PCR purification kit. Hairpin structure 1 (5'-AATT GCGAG TTG CGA GTT GTA AAA CGA CGG CCA GT CTCGC) was formed by heating to 50°C, following by cooling, that allowed the oligonucleotide to self-anneal at the underlined sequences, yielding a 5'-AATT overhang. The loop structure contained the M13 universal primer sequence. Hairpin structure 1 and pUC18 PCR product were combined in a 10:1 molar ratio, respectively, and treated with five units $T_4$ polynucleotide kinase at 37°C for 40 min, ligated with 400 cohesive end units of $T_4$ DNA ligase at 16°C for 30 min, and then inactivated at 65°C for 10 min to form a dumbbell template. When denatured, the dumbbell template becomes a single-stranded circle. The dumbbell template was purified with QIAquick PCR cleanup kit to remove the excess, unligated hairpin structures.

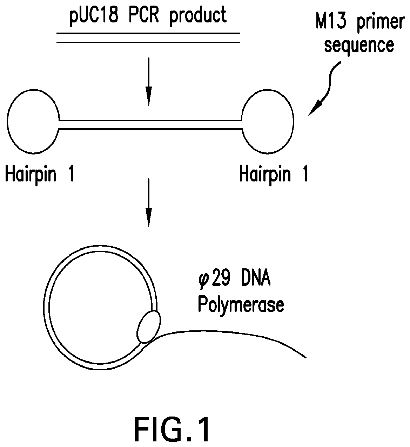

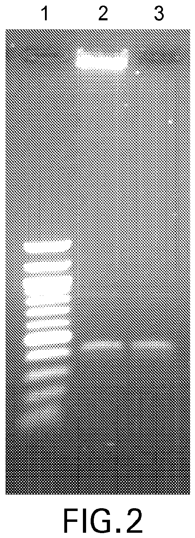

Rolling circle replication was performed on the dumbbell template using the reverse complement (RC) of the M13 primer as illustrated in FIG. 1. Here, 2 $\mu$M of M13-RC primer (5'-ACT GGC CGT CGT TTT ACA A) and M13 control primer (5'-TTG TAA AAC GAC GGC CAGT) were separately annealed to -10 ng of the pUC18 dumbbell template by heating to 94°C for 5 min. and cooling to 57°C for 1 min. in $\varphi$29 reaction buffer with 200 $\mu$M dNTPs and 200 $\mu$g/mL BSA in a 20$\mu$L reaction. The reaction was cooled further to 30°C, whereupon 10 units of $\varphi$29 DNA polymerase were added to the primed dumbbell templates and incubated for 30 min., followed by heat-inactivation at 65°C for 10 min. As a control, the normal M13 universal sequencing primer was incubated in a separate rolling circle replication mixture. The replicated dumbbell templates were then analyzed by gel electrophoresis. As shown in FIG. 2, the M13-RCR product (lane 2) yielded a high molecular weight product (upper band in the well), whereas the M13 control yielded no visible rolling circle replication product. The results in lane 2 are evident of the rolling circle replication method creating high molecular weight dumbbell templates.

The method shown above is just one manner in which to attach hairpin structures to the ends of fragmented nucleic acid molecules. For example and without limitations, hairpin structures can also be attached by TA-cloning and blunt end ligation, such as Hairpin structure 2 (5'-T GCGAG TTG CGA GTT GTA AAA CGA CGG CCA GT CTCGC) with a "T"-overhang. The 425 bp pUC18 PCR amplicon may also be treated with the NEBNext dA-tailing kit to attach a "dA" residue at the 3'-ends of the DNA fragments. The pUC18 amplicon and Hairpin structure 2 then will be phosphorylated and ligated together using the method described above to create the dumbbell template. This approach integrates into the majority of next-generation sequencing library construction methods for whole genome samples.

Example 2

Genomic DNA can also be used as a starting sample. For example and without limitation, purified genomic DNA from HapMap sample NA18507 can be obtained from Coriell Cell Repositories and sheared using standard next-generation sequencing methods (*i.e.,* using a Covaris E210R device) and then size-selected for fragments in size increments of 0.5, 1.0, 2.5, 5.0, 7.5, and 10.0 kb. Similar to that described above, the DNA sample can be subjected to fragmenting to produce the different size DNA fragments, quantification of the starting number of fragments, ligation of hpA/hpB using identical conditions, and enrichment for hpA-fragment-hpB dumbbell templates. The enrichment factor would be determined using dual-labeled fluorescence microscopy to enumerate colocalized fluorescent signals and comparing that number to the total number of fluorescent signals. The Nikon Eclipse microscope analytical tools can perform a number of analyses, including intensity measurements, colocalization of multiple fluorescent signals, and others as included in the core package items.

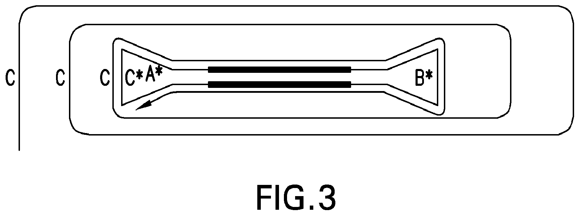

In this example, 500 ng of normal human genomic DNA (Millipore) was digested with *Eco*R1 in a dilute restriction enzyme reaction, followed by inactivation at 65°C. These fragments, containing a 5'-AATT overhang at either end, were ligated to stable and unique hairpin structure, HP1. HP1 (5'-AATT GCGAG TTG CGA GTT GTA AAA CGA CGG CCA GT CTCGC) was formed by heating to 95°C in a high salt buffer, followed by rapid cooling on ice, that allowed the oligonucleotide to self-anneal at the underlined sequences, yielding a 5'-AATT overhang. HP1 and digested genomic DNA were combined in a 10:1 molar ratio, respectively, and ligated with 400 cohesive end units of $T_4$ DNA ligase at 16°C for 30 min, and then inactivated at 65°C for 10 min to form a dumbbell template, see FIG. 3. The dilute digestion and HP1 ligation generated a smear of dumbbell template DNA ranging in size from approximately 20 kb to 1 kb. The dumbbell templates were gel purified to remove excess unligated and self-ligated HP1 adapters and size selected to isolate three different fragment sizes, 10-6 kb, 6-3 kb, and 3-2 kb.

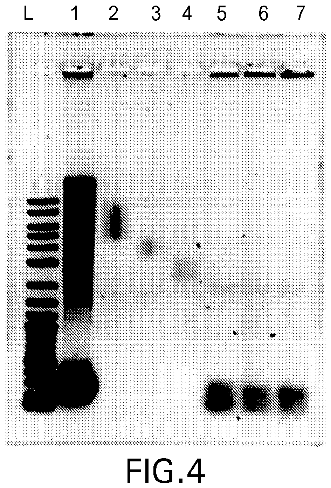

The loop structure of HP1 contained the M13 universal primer sequence. RCR *(i.e.,* using one primer) was performed on the dumbbell template using the reverse complement (RC) of the M13 primer, see FIG. 3. Here, 2 μM of M13-RC primer and M13 control primer (not shown) were separately annealed to -10 ng of the size-selected genomic DNA dumbbell templates by heating to 94°C for 5 min and cooling to 45°C for 2 min in φ29 reaction buffer with 200 μM dNTPs and 200 μg/mL BSA in a 20μL reaction. The reaction was cooled further to 30°C, whereupon 10 units of φ29 DNA polymerase were added to the primed circles and incubated for 60 min., followed by heat-inactivation at 65°C for 10 min. The starting and end materials were analyzed by agarose gel electrophoresis. As shown in FIG. 4, *Eco*R1 digested, HP1 ligated genomic DNA was loaded in the well of Lane 1. Size selected and purified dumbbell templates were loaded in wells of Lanes 2, 3, and 4. The RCR products, loaded in Lanes 5, 6, and 7, appear to be immobile complexes remaining in the wells following gel electrophoresis. The expected M13-RC RCR products yielded a high molecular weight products (upper band in the wells of lanes 5, 6, and 7 of FIG. 4), whereas the M13 control yielded no visible RCR product (not shown). The results in lanes 5, 6, and 7 of FIG. 4 are evident of the RCR method using a dumbbell template to create high molecular weight DNA.

Example 3

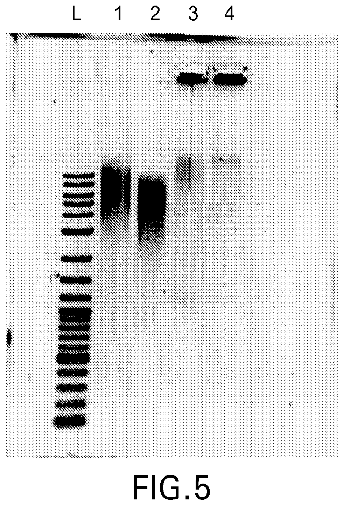

Replicating dumbbell templates were also created from large fragmented, dA-Tailed genomic DNA. Here, hairpins were attached by TA-cloning and blunt end ligation. The TA-cloning approach integrates nicely into the majority of current NGS platforms. We have designed Hairpin 2 (HP2) (5' -/Phos-CTTTTTCTTTCTTTTCT GGGTTGCGTCTGTTCGTCT AGAAAAGAAAGAAAAAGT) with a "T"-overhang. Human genomic DNA (500 ng) was fragmented using the Covaris G-tube to achieve tightly defined fragment length populations, as shown in Lanes 1 and 2 of FIG. 5. This genomic DNA was then end-repaired and dA-Tailed using the End-Preparation Module of the NEBNext Ultra DNA Library Prep Kit. HP2 was self-annealed similar to HP1, and ligated to the repaired genomic DNA using Blunt/TA Ligase Master Mix (5:1 molar ratio). Excess HP2 and unligated genomic DNA were removed using Exonucleases III and VII.

The resulting dumbbell templates were purified using Qiaex ii beads and an RCR reaction using a unique primer (5'-AAAAAAA CAGACGCAACCC) was carried out similar to the previously described reaction. As shown in FIG. 5, the two fragmented genomic DNA populations display the highly tunable fragmentation capacity of the Covaris G-tube (Lanes 1 and 2). The RCR products resulting from the end-repaired, dA-Tailed, HP2 ligated fragments remain as highly immobile complexes remaining in the wells following gel electrophoresis (Lanes 3 and 4).

Example 4

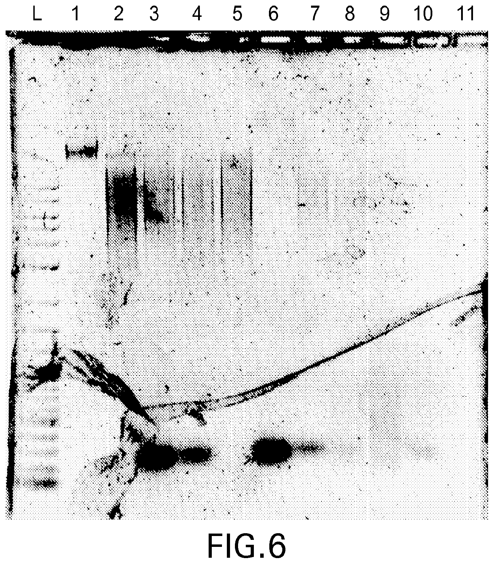

In another experiment, about 20 μL high molecular weight, normal human genomic DNA (gDNA) (100 ng/μL) was combined with 130 μL HPLC grade $H_2O$, and the total 150 μL was pipetted onto into Covaris G-Tube. The tube was first centrifuged at 5600 RCF *(i.e.,* relative centrifugal force) for 1 minute, and then the orientation of the G-Tube was reversed and centrifuged at 5600 RCF for 1 minute. This yielded approximately 8-10 kilobase fragments of genomic DNA, as shown in lane 2 of FIG. 6.

Genomic DNA samples can also be fragmented using several methods known in the art, including but not limited to, enzymatic fragmentations using New England Biolabs (NEB) Fragmentase, nucleases, and restriction enzymes; fragmentations using mechanical forces such as needle shearing through small gauge needles, sonication, point-sink shearing, nebulization, acoustic fragmentation, and transposome mediated fragmentation.

The ends of the fragmented DNA are then prepared for ligation with the appropriate adaptors using one of several means such as removal or incorporation of nucleotides at overhanging 5'- and 3'-ends, 5' phosphorylation, and dA-Tailing. About 55.5 μL of fragmented gDNA in $H_2O$ as prepared above is combined with 6.5 μL of 10x End-Repair Buffer (NEB) and 3 μL of End-Preparation Enzyme Mix (NEB) and aliquoted into a thermocycler microtube. This reaction mixture is then incubated at 20°C for 30 minutes, followed by 65°C for 30 minutes. The reaction is chilled and prepared for the next steps by placing the reaction tube on ice or at 4°C.

Hairpin adapters were created from linear oligonucleotides. Lyophilized adapters were reconstituted to 100 μM in HPLC $H_2O$. The following components were combined in a micro centrifuge tube: 10 μL of 100 μM Adapter Stock, 5 μL of 10x End-Repair Buffer (NEB), 1 μL of 500 mM NaCl, and 34 μL of HPLC $H_2O$. The mixture was incubated at 95°C for 15 minutes and then immediately moved to 4°C.

The dumbbell templates were created by attachment of hairpin adapters on each end of the fragmented, end-repaired gDNA. The following components were combined to form a sample reaction mixture: 65 μL of fragmented gDNA with repaired-ends as described above, 3 μL of 20 μM adapters prepared as described above, 15 μL of Blunt/TA ligation Master Mix (NEB) and 3 μL of HPLC $H_2O$. This ligation reaction was allowed to proceed at 20°C for 1 to 16 hours and then immediately moved to 4°C.

The unligated adaptors and fragmented DNA were subject to an exonuclease digestion. The following components were combined to form a sample reaction mixture: 1 $\mu$L of 10x Exonuclease VII Buffer (NEB), 1 $\mu$L of Exonuclease VII, 1 $\mu$L of Exonuclease III, and 7 $\mu$L of HPLC H$_2$O. This mixture was added to the ligation reaction mixture containing the dumbbell templates, the unligated adaptors, and the fragmented DNA with free ends. The resulting reaction mixture was incubated at 37°C for 1 hour, then at 95°C for 10 minutes; then transferred back to 4°C.

FIG. 6 is an example of an agarose gel analysis of DNA products prepared as described above. Lane 1 shows unfragmented genomic DNA; Lane 2 shows fragmented DNA following fragmentation in a Covaris G-Tube; Lane 3 shows products formed following ligation of adaptors to 1 $\mu$g of fragmented DNA; Lane 4 shows products formed following ligation of adaptors to 500 ng of fragmented DNA; Lane 5 shows products formed following 1 $\mu$g of fragmented DNA in the ligation reaction without any adaptors; Lane 6 shows products formed following a ligation reaction with no fragmented DNA and only the adaptors; Lane 7 shows products formed following exonuclease digestion of products obtained from ligation of adaptors to 1 $\mu$g of fragmented DNA; Lane 8 shows products formed following exonuclease digestion of products with Exo III and Exo VII obtained from ligation of adaptors to 500 ng of fragmented DNA; Lane 9 shows products formed following exonuclease digestion of fragmented DNA with Exo III and Exo VII in the ligation reaction without any adaptors; Lane 10 shows products formed following exonuclease digestion of products with Exo III and Exo VII obtained from a ligation reaction with no fragmented DNA and only the adaptors. Lane 11 shows a digestion control of fragmented genomic DNA and adapters that were not ligated.

The DNA samples were also subject to concentration to remove salts and concentrate exonuclease resistant dumbbell templates. The volume of the reaction mixture after the exonuclease digestion was adjusted with about 4 $\mu$L HPLC H$_2$O to 100 $\mu$L solution. About 10 $\mu$L of 3 M sodium acetate at pH 5.2 and 5 $\mu$L Glycogen (20 mg/mL) were added to the solution, followed by the addition of 115 $\mu$L cold 100% Isopropanol. This reaction mixture was refrigerated at -20°C for >1 hour, and then centrifuged at 10 RCF for 20 minutes at room temperature. The supernatant was aspirated and the precipitate was washed with 70% ethanol. The final precipitate was allowed to dry for -15 minutes, and then resuspended in 30 $\mu$L of 10 $\mu$M Tris-HCl, pH 8.0.

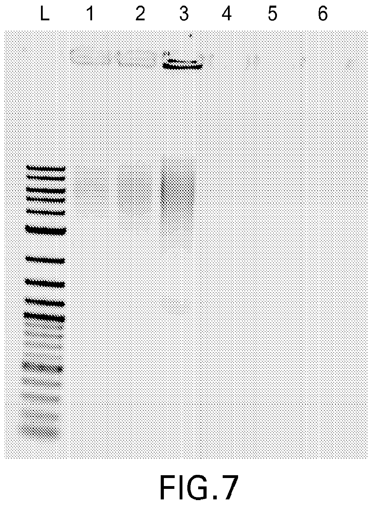

FIG. 7 is an example of an agarose gel analysis of DNA products prepared as described above. Lane 1 shows products formed following ligation of adaptors 10.1 to fragmented DNA and subsequent ethanol precipitation; Lane 2 shows products formed following ligation of adaptors 2.1 to fragmented DNA and subsequent ethanol precipitation; Lane 3 shows products formed following fragmented DNA in a ligation reaction with no adaptors and subsequent ethanol precipitation; Lane 4 shows no products were formed following only adaptors 10.1 in a ligation reaction with no fragmented DNA and subsequent ethanol precipitation; Lane 5 shows no products were formed following only adaptors 2.1 in a ligation reaction with no fragmented DNA and subsequent ethanol precipitation; Lane 6 shows no products were formed following a ligation reaction with no fragmented DNA and adaptors and subsequent ethanol precipitation.

The dumbbell templates were also subject to size selection. Exonuclease resistant dumbbell templates of desired size were isolated by agarose gel electrophoresis to minimize carryover of any undesired products such as adapter-adapter ligated products. A 0.8% (weight/volume) 1x TAE agarose gel was prepared. The concentrated dumbbell templates with appropriate amounts of DNA loading dye were prepared and about 20 $\mu$L of concentrated dumbbell templates were loaded onto the agarose gels. After sufficient time for separation of the products, the gels were stained with SybrSafe gel stain, and visualize on a light box. Using a sterile scalpel, sections of the gel containing the desired size range of the dumbbell templates were excised. The dumbbell templates were isolated using the Qiaex ii isolation protocol and resuspended in 30 $\mu$L H$_2$O.

The dumbbell templates were then subject to a rolling circle replication using highly processive, strand-displacing polymerases. A first reaction mixture was set up with the following components: 5 $\mu$L of size-selected dumbbell templates, 1.5 $\mu$L of 10x phi29 polymerase buffer (NEB), 1 $\mu$L of dumbbell complementary primer, 0.5 $\mu$L of Bovine Serum Albumin (BSA) - 100mg/mL, and 7 $\mu$L of HPLC H$_2$O. The reaction mixture was incubated at 95°C for 10 minutes, cooled to 45°C for 5 minutes, and then further cooled to 20°C. A second reaction mixture was set up with the following components: 1 $\mu$L of 10x phi29 polymerase buffer (NEB), 5 $\mu$L of 10 mM dNTP Mix, 0.5 $\mu$L of phi29 polymerase, and 3.5 $\mu$L of HPLC H$_2$O. The first reaction mixture post processing as described above and the second reaction mixture were combined and incubated at 25°C for 1-4 hours. Then, the resulting mixture was heated to 65°C for 20 minutes to inactivate the polymerase.

The rolling circle replication products were then analyzed by agarose gel electrophoresis. Due to their high molecular weight, these rolling circle replication products were present in the well and did not enter the gel following electrophoresis. Certain additional early termination products are also visible.

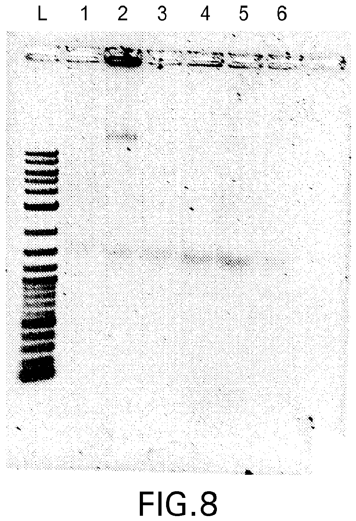

FIG. 8 is an example of an agarose gel analysis of DNA products prepared by rolling circle replication of products analyzed in FIG. 6. Lane 1 shows an inefficient rolling circle reaction of products excised from Lane 7 of FIG. 6. Lane 2 shows the rolling circle products obtained after rolling circle replication of products excised from Lane 8 of FIG. 6 as a highly immobile complex remaining in the wells following gel electrophoresis. Lane 3 shows no rolling circle products were obtained after rolling circle replication of products excised from Lane 9 of FIG. 6. Lane 4 shows no rolling circle products were obtained after rolling circle replication of products excised from Lane 10 of FIG. 6. Lane 5 shows no rolling circle products were obtained from the rolling circle reaction with no DNA present. Lane 6 shows no rolling circle products were obtained from rolling circle reaction with the fragmented DNA products, showing that there is no random priming from the fragmented DNA.

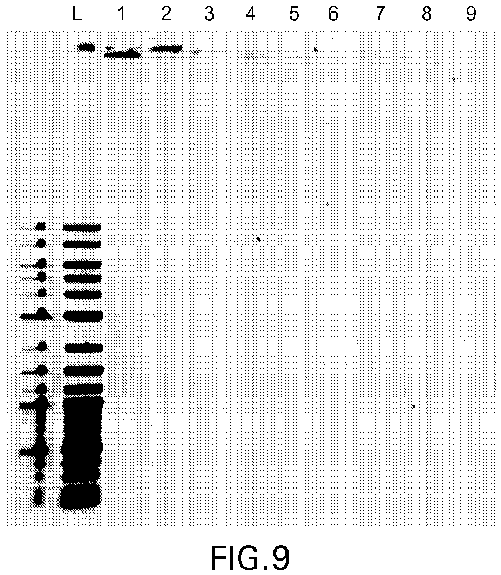

FIG. 9 is an example of an agarose gel analysis of DNA products prepared by rolling circle replication of products analyzed in FIG. 7. Lane 1 shows the rolling circle products obtained after rolling circle replication of size-selected products analyzed in Lane 1 of FIG. 7. The immobile complex remaining in the wells following gel electrophoresis is indicative of a successful RCR product. Lane 2 shows the rolling circle products obtained after rolling circle replication of size selected products analyzed in Lane 2 of FIG. 7. Lane 3 shows no rolling circle products were obtained after rolling circle replication of size selected products analyzed in Lane 3 of FIG. 7. Lane 4 shows no rolling circle products were obtained after rolling circle replication of size selected products analyzed in Lane 4 of FIG. 7. Lane 5 shows no rolling circle products were obtained after rolling circle replication of size selected products analyzed in Lane 5 of FIG. 7. Lane 6 shows no rolling circle products were obtained after rolling circle replication of size selected products analyzed in Lane 6 of FIG. 7. Lanes 7, 8, and 9 show no rolling circle products were obtained in the control reactions where fragmented DNA were provided to a rolling circle reaction without ligation (Lane 7), fragmented DNA were provided to a rolling circle reaction without primers (Lane 8), and no fragmented DNA was provided to a rolling circle reaction without ligation (Lane 9).

Example 5

The rolling circle replication products can also be detected by using molecular probes or beacons directed toward complementary regions of the hairpin sequence of the dumbbell templates. To demonstrate the feasibility of this method, a titration series of H3 hairpin adapters was created with concentrations ranging from 0 $\mu$M to 5 $\mu$M. A stock solution of 10 $\mu$M H3 hairpin was serially diluted to achieve 2x testing concentrations. Hairpin adaptor 3 (H3) has the following sequence:

5'PO$_4$–AATTG CGAGC TATGA CCATG ATTAC GCCAC TGGCC GTCGT TTTAC AACTC GC

For example, the 10 $\mu$M stock was diluted in half to achieve a 5 $\mu$M test sample, the 5 $\mu$M stock was diluted in half to achieve a 2.5 $\mu$M test sample, and so on. These represent twice (2x) the actual test concentration. About 5 $\mu$L of the 2x H3 adapter concentrations were then combined with 1 $\mu$L of NEB phi29 Reaction Buffer 10x, 1 $\mu$L of 200 $\mu$M Beacon 2, and 3 $\mu$L of HPLC H$_2$O. Molecular Beacon 2 has the following sequence, and "5,6-FAM" is a mixture of 5-FAM and 6-FAM isomers and "IABkFQ" is an IowaBlack quencher:
5'-/5,6-F AM/CGGAGTTGCGAGTTGTAAAACGACGGCCAGTCTCCG/3-IABkFQ

In setting this reaction mixture, the H3 concentration in the test sample was reduced to the final 1x measured concentration. The reaction mixtures were then heated to 98°C on a hotplate, maintained at that temperature for ten minutes, and then allowed to cool slowly on the benchtop. All the reactions and thermocycling steps were carried out with the lights off and with reaction tubes covered in tinfoil to prevent loss of signal from the beacon. Once cooled to room temperature, the reactions were prepared for reading on a Molecular Devices SpectraMax Gemini XPS fluorescent microplate reader. Specifically, the SpectraDrop Microplate Slide was used to facilitate measurement of very small volumes. About 2 $\mu$L from each titration reaction was loaded on to the micro-volume slide. Once inserted into the machine, the following program was run at room temperature:

Excitation wavelength: 495 nm
Emission wavelength: 520 nm
6 Flashes/read

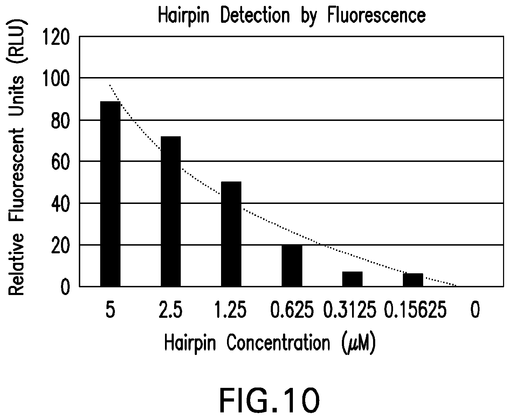

The raw data was collected and processed as shown in FIG. 10. The RLU (relative luminescence units) reading for 0 $\mu$M was subtracted from all samples to normalize by eliminating background fluorescence.

*Table 1*

| uM [H3] | 5 | 2.5 | 1.25 | 0.625 | 0.3125 | 0.15625 | 0 |
|---|---|---|---|---|---|---|---|
| RFU | 134.896 | 118.257 | 96.351 | 66.297 | 53.12 | 52.34 | 46.489 |
| Adjusted | 88.407 | 71.768 | 49.862 | 19.808 | 6.631 | 5.851 | 0 |

Example 6

Experiments can be designed to determine the efficiency of making dumbbell templates independent of fragment length size. A major limitation of current large-fragment NGS library construction methods is creating mate-pair templates by circularizing the ends of long DNA fragments. Ideally, the efficient ability to create dumbbell templates should be size independent. These dumbbell templates may be of various sizes, including for example without limitation, of 5.0, 7.5, or 10.0 kb. These fragment sizes first may be created with PCR by designing primers using human BAC DNA that target the same genomic region. This approach will allow the use of real-time PCR to quantify the copy number of the different size dumbbell templates. In an example, real-time PCR reagents for the *TCF7L2* rs7903146 allele have been created, which were designed using the Life Technologies custom TaqMan assay website. The 5'-primer sequence was 5'-CCT CAA ACC TAG CAC AGC TGT TAT, the 3'-primer sequence was 5'-TGA AAA CTA AGG GTG CCT CAT ACG, and the probe sequence was 5'-CTT TTT AGA TA[C/T] TAT ATA ATT TAA. In other examples, one could produce the different size fragments, quantify the starting number of amplicons, ligate Hairpin structure 2 using identical conditions, and then quantify the dumbbell template copy number with real-time PCR.

In other experiments, defined fragment populations can be created primarily through two methods, the Covaris G-Tube and NEB Fragmentase. Preparation and isolation of dumbbell templates will follow according to the TA-cloning methods described herein. Molecular beacons that hybridize with hairpin sequences can be used to quantify the number of dumbbell templates of different size using a fluorescent plate reader. These experiments demonstrate that dumbbell templates can be created efficiently, independent of the fragment size of the starting genomic DNA sample. Furthermore, these molecular beacons can also be used to quantify RCR products and the reaction efficiency.

Example 7

Efficient insertion of dumbbell templates in NGS paired-end sequencing platforms requires the presence of unique primers or hairpins on the each end of a DNA template. This will be accomplished through standard end repair/ dA-tailing methods followed by the ligation of two unique hairpin oligonucleotides (i.e., hpA and hpB), each containing unique universal replication/sequencing priming and molecular beacon sites. Following hairpin ligation, we expect a population composed of 25% hpA-fragment-hpA, 50% hpA-fragment-hpB, and 25% hpB-fragment-hpB. The desired form, hpA-fragment-hpB, may be enriched by capture-probe chromatography by first passing the ligation product through a column containing the reverse complement of Hairpin A, thus capturing the hpA-fragment-hpA and hpA-fragment-hpB templates, but not the hpB-fragment-hpB templates. Following elution, the partially enriched sample is then passed over a second column containing the reverse complement of Hairpin B thus capturing the hpA-fragment-hpB, but not the hpA-fragment-hpA templates. This dual-hairpin approach will be demonstrated using similar approaches as outlined above; populations of uniquely sized DNA fragments centered around 5.0, 7.5, and 10.0 kb will be created, size selected, purified, end-repaired and dA-Tailed. These will then be ligated to hpA and hpB hairpins and dual-labeled, hpA-fragment-hpB dumbbell templates will be enriched using aforementioned techniques. Initial experiments using φ29 DNA polymerase and the $T_7$ replisome system will be performed to assess the dependence of replication copy number on dumbbell template size using the solution-based molecular beacon.

Example 8

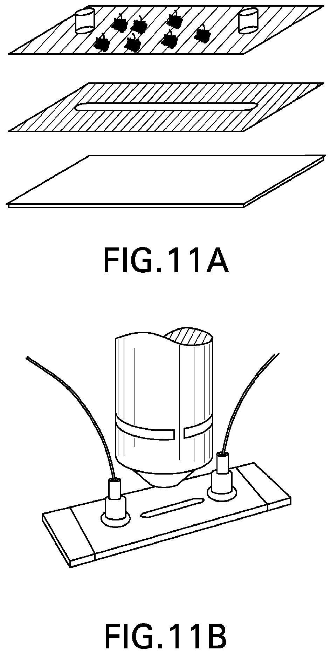

Conditions for the rolling circle amplification can be optimized to include appropriate DNA polymerases, replication factors, and reaction conditions such that at least a 1,000-fold replication of 10 kb dumbbell templates can be supported. A replication fold of 1,000 copies is targeted as this is the equivalent number of clonally-amplified short templates achieved on an Illumina cBot instrument, and therefore, one can expect similar levels of fluorescent signals measured during the sequencing process. A panel of DNA polymerases can be adopted for long synthesis, including commercially available DNA polymerases (φ29, LongAmp, *Bst, Bst* 2.0, Q5, and $T_7$ DNA polymerases) as well as at least 12 noncommercial, proprietary Family A, B, and D DNA polymerases. In addition, a panel of replication accessory factors can also be used as replication enhancers. Accessory proteins can be added to increase efficiency of production of desired rolling circle products, including but not limited to, the processivity clamp and clamp loader complex to increase DNA polymerase processivity, single stranded binding proteins to stabilize single-stranded DNA regions, helicases to separate double-stranded DNA ahead of the DNA polymerase, flap endonuclease for resolving flap DNA structures, and DNA ligase to seal DNA nicks. These factors are interchangeable with DNA polymerases from within the same Family and may be tested with appropriate DNA polymerase partners. For example, core accessory factors from the archaeon *Thermococcus* sp. 9°N will be used with Family B DNA polymerases while Family A DNA polymerases will be tested with *E. coli* accessory factors. Quantitative PCR will be used to measure replicated dumbbell template DNA. A qPCR probe can target the hairpin region of the 10 kb dumbbell templates created as described herein. With replication of the primed dumbbell template, the probe can bind each segment of the synthesized hairpin region. Probe intensity can therefore be used to indicate copy number when compared to a standard series of diluted hairpin templates (FIG. 11). In addition to qPCR, the length of amplification products can be monitored by alkaline agarose gel electrophoresis. Alkaline agarose gel electrophoresis separates DNA into single strands and accurately measures the overall replication product length.

Example 9

In an example, an optimal density of functionalized primers for rolling circle replication is attached to a glass surface of a custom-designed flowcell. A custom cut adhesive gasket sandwiched between two glass slides was designed as shown in FIG. 11A. Replicons are attached to the bottom side of a coverslip. The glass coverslip has inlet/outlet ports fastened with nanoport fittings. The gasket here is a 3M Double-sided tape with a microchannel, and lies on top of a standard microscope slide. This design pairs the requisite optical, chemical, and mechanical properties with practical necessities like ease of use, speed of fabrication, simplicity, and cost effectiveness.

As shown in FIG. 11B, a flowcell design consists of a microchannel formed by sandwiching a 130 $\mu$m thick 3M double-sided adhesive film gasket between a standard 1 mm thick 25 x 75 mm borosilicate glass slide (VWR) and a 25 x 75 mm #1.5H borosilicate coverslip (Schott Nexterion). The microfluidic channel gasket layer was cut out of 3M double-sided adhesive tape using a laser-cutter (Universal X-660), and inlet/outlet holes were sand-blasted through the top coverslip layer. The channel was sealed by placing the adhesive gasket on top of the glass slide and then placing a coverslip on top of the gasket. The resulting channel has a rectangular cross section 130 $\mu$m deep, 3 mm wide, and 4 cm long. Nanoport fixtures (IDEX Health & Science) were used to connect 100 $\mu$m ID PEEK tubing to the inlet and outlet ports as a means for exchanging solutions and reagents within the flowcell.

The pre-synthesized oligonucleotides may be attached to the glass surfaces by use of chemical strategies. Identifying optimal support chemistry is important as previous studies have shown that certain coupling strategies can impact the performance of hybridization and solid-phase PCR applications. In an example, functionalization of the glass surface with a silane reagent, such as 3-aminopropyltriethoxysilane is a first step. Many chemical coupling strategies involve amino-modified oligonucleotides. Using these end-functional groups as a starting point permits the use of a systematic approach to evaluate different intermediate coupling agents for the attachment of oligonucleotides to a glass surface. For example, and without limitation, the cyanuric chloride activation method has been used to attach the oligo-nucleotide sequence 5'-NH$_2$-TTTTTTTTTTTTGTAAAACGACGGCCAGT to the coverslip surface. Other examples may utilize several other activation chemistries, for example, the 1,4-phenylene diisothiocyanate and the dicarboxylic acid reactions. All these activation strategies yield similarly good hybridization data. Embodiments of the invention include the poly(dT)n linkers of different lengths (*i.e.,* n = 0, 10, 20).

In an example, one can utilize Nikon Eclipse FN1 microscope that uses a broadband LED light source and provides flexibility with different fluorescent dyes that span the visible and near-IR regions. In an example, the pUC18 dumbbell template was created using the dA-tailing method with Hairpin structure 3 (5'-T CGCGAG CTATGACCATGATTACGCC ACTGGCCGTCGTTTTACAA CTCGCG). Molecular beacon 2 (5'-FAM-CGGAG CTATGACCATGATTACGCC CTCCG-IowaBlack) has been designed to assay for solid-phase rolling circle replication reactions in the above-described flowcell. The underlined sequences represent the double-stranded stem region, the first boxed sequence represents the probe sequence, and the second boxed sequence represents the primer sequence that will bind to the immobilized M13 primer sequence. As molecular beacons should yield low background fluorescence, good signal-to-noise ratios (SNRs) will be generated with sufficient rolling circle replication generating surface-bound replicons. Dilutions of 0.5-kb dumbbell templates may be determined empirically to target a replicon density of 25-to-50k per field of view (FOV).

In certain examples, surface effects might inhibit some reactions and may require the use of passivating agents, such as polyvinylpyrrolidone or high molecular weight PEG. In certain examples, low yield, phosphorothiolate primers may be utilized as $\varphi$29 DNA polymerase can exhibit significant exonuclease activity with single-stranded DNA.

Example 10

The reagents and conditions gleaned from the previous examples can be applied to fragmented human genomic DNA to demonstrate the robust ability to create NGS compatible, clonally-replicated clusters from 10 kb dumbbell templates. In certain examples, the 10-kb dumbbell templates can yield 1,000 copies of target sequence. Several DNA polymerases work efficiently in the rolling circle replication method, including but not limited to $\varphi$29, *Bst,* and Vent(exo-) DNA polymerases. Recently, a mutant $\varphi$29 DNA polymerase was identified to increase DNA synthesis yields by several-fold and is commercially-available from Sygnis, Inc. In certain examples, replisome complexes can be used to replicate 10 kb dumbbell templates using the coordinated activities of T$_7$ Sequenase, T$_7$ helicase, and T$_7$ single-stranded binding protein. In certain examples, the dumbbell templates (in size increments of 5.0, 7.5, and 10.0 kb) may be used for solution assays and analyzed using the real-time PCR test for *TCF7L2*. In certain examples, one may include accessory proteins, including for example without limitation, other helicases, single-stranded binding proteins, thioredoxin, topoisomerases, reverse gyrases, or any combinations thereof, to improve the efficiency and accuracy of rolling circle replication method.

In certain examples the dumbbell templates of 5.0, 7.5, or 10.0 kb can be created with the Hairpin structure 3 and tested with several of the optimal conditions identified in the solution-based real-time PCR assay. Following the rolling circle replication method, the replicated dumbbell templates may be probed with Molecular Beacon 2 and analyzed by fluorescence microscopy to determine signal intensities of the replicated dumbbell templates. In certain examples, the rolling circle replication may be performed with the dual-hairpin dumbbell templates as real-world templates isolated from HapMap sample NA18507.

Disclosures of the present application can also include one or more hairpin structures, enzymes, other nucleotide and protein reagents packaged as kits for practicing the methods and producing the compositions described herein. Reagents for use in practicing methods and detecting the presence of rolling circle products as described herein can be provided individually or can be packaged together in kit form. For example, kits can be prepared comprising one or more primers, one or more labeled nucleoside triphosphates, and associated enzymes for carrying out the various steps of the methods described herein. Kits can also include packaged combinations of one or more affinity labeled hairpin structures and corresponding solid support(s) to purify the dumbbell templates. The arrangement of the reagents within containers of the kit will depend on the specific reagents involved. Each reagent can be packaged in an individual container, but various combinations may also be possible. Disclosures of the present application can also include a kit containing one or more oligonucleotides to form one or more hairpin structures, a set of components for ligation, including ligases, cofactors, accessory factors, and appropriate buffers, and a set of components for replication including substantially complementary primers, enzymes that perform various steps described herein, accessory factors, and appropriate buffers.

Certain disclosures of the present application include a kit containing at least one oligonucleotide capable of forming a hairpin structure; a first set of components for ligating the hairpin structure to at least one nucleic acid molecule from a sample to form at least one dumbbell template, wherein the first set of components contain one or more of a ligase, cofactors, a ligase-appropriate buffer, and combinations thereof; a second set of components for purifying the at least one dumbbell template by digesting any unligated hairpin structure and any unligated nucleic acid molecule, wherein the second set of components contain one or more of a an exonuclease, an exonuclease-appropriate buffer, and combinations thereof; and a third set of components for replicating the at least one dumbbell template to form at least one amplified dumbbell template, wherein the third set of components contain a polymerase or a replisome, nucleotides, accessory factors, and at least one primer substantially complementary to a region of the at least one dumbbell template.

Disclosures of the present application also include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a polymerase and at least one primer substantially complementary to a region of the at least one dumbbell template for replicating the at least one dumbbell template to form at least one replicated dumbbell template.

Certain disclosures of the present application include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a replisome and at least one primer substantially complementary to a region of the at least one dumbbell template for replicating the at least one dumbbell template to form at least one replicated dumbbell template.

Certain disclosures of the present application include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a polymerase and at least two primers substantially complementary to at least two regions of the at least one dumbbell template for amplifying the at least one dumbbell template to form at least one amplified dumbbell template.

Certain disclosures of the present application include a kit containing at least one oligonucleotide capable of forming a hairpin structure; a ligase for ligating the hairpin structure to at least one nucleic acid molecule from a sample to form at least one dumbbell template; an exonuclease for purifying the at least one dumbbell template by digesting any unligated hairpin structure and any unligated nucleic acid molecule; and a replisome and at least two primers substantially complementary to at least two regions of the at least one dumbbell template for amplifying the at least one dumbbell template to form at least one amplified dumbbell template.

Moreover, the foregoing has broadly outlined certain objectives, features, and technical advantages of the present invention and a detailed description of the invention so that embodiments of the invention may be better understood in light of features and advantages of the invention as described herein, which form the subject of certain claims of the invention. It should be appreciated that the conception and specific embodiments disclosed may be readily utilized as a basis for modifying or designing other structures for carrying out the same purposes of the present invention. It should also be realized that such equivalent constructions do not depart from the invention as set forth in the appended claims. The novel features which are believed to be characteristic of the inventions, both as to its organization and method of operation, together with further objects and advantages are better understood from the description above when considered in connection with the accompanying figures. It is to be expressly understood, however, that such description and figures are provided for the purpose of illustration and description only and are not intended as a definition of the limits of the present invention. It will be apparent to those skilled in the art that various modifications and changes can be made.

**Claims**

1. A method of replication or amplification of at least one nucleic acid molecule, the method comprising:

(a) ligating hairpin structures to each end of at least one double-stranded nucleic acid molecule with a minimum length of greater than 5 kb isolated from a sample to form at least one dumbbell template;
(b) contacting said at least one dumbbell template with at least one substantially complementary primer in a method of replication or with at least two substantially complementary primers in a method of amplification, wherein said at least one substantially complementary primer is attached to at least one substrate; and
(c) performing rolling circle replication on said at least one dumbbell template contacted with the at least one substantially complementary primer to form at least one replicated or amplified dumbbell template.

2. The method of claim 1, wherein the double-stranded nucleic acid molecule has a length greater than 10 kb.

3. The method of claim 1, wherein the double-stranded nucleic acid molecule is at least 20 kilobases long.

4. The method of any of claims 1 to 3, wherein:
prior to the ligating step, the method further comprises fragmenting the at least one nucleic acid molecule to form at least one fragmented nucleic acid molecule with a minimum length of greater than 5 kb, greater than 10 kb or at least 20 kilobases long.

5. The method of amplification according to any of claims 1 to 3 wherein:
prior to the contacting step, the method further comprises purifying said at least one dumbbell template by treating any unligated hairpin structure and any unligated nucleic acid molecule with an exonuclease.

6. A method of detecting at least one replicated or amplified dumbbell template, the method comprising:

(a) fragmenting at least one nucleic acid molecule to form at least one fragmented nucleic acid molecule with a minimum length of greater than 5 kb, greater than 10 kb or at least 20 kilobases long;
(b) replicating or amplifying the fragmented nucleic acid molecule according to the replication method of claim 1; and
(c) detecting said at least one replicated or amplified dumbbell template.

7. The method of Claim 6, wherein the step of detecting said at least one replicated or amplified dumbbell template consists of sequencing said at least one replicated or amplified dumbbell template.

8. The method of Claim 6, wherein the step of detecting said at least one replicated or amplified dumbbell template comprises contacting said at least one replicated or amplified dumbbell template with a DNA probe.

9. The method of claim 6, wherein:
prior to the contacting step, the method further comprises purifying said at least one
dumbbell template by treating any unligated hairpin structure and any unligated fragmented nucleic acid molecule with an exonuclease.

**Patentansprüche**

1. Verfahren für eine Replikation oder eine Amplifikation wenigstens eines Nukleinsäuremoleküls, wobei das Verfahren umfasst:

(a) Ligieren von Haarnadelstrukturen an jedes Ende wenigstens eines doppelsträngigen Nukleinsäuremoleküls mit einer Mindestlänge von über 5 kb, das aus einer Probe isoliert wird, um wenigstens eine Hantelmatrize auszubilden;

(b) Inberührungbringen der wenigstens einen Hantelmatrize mit wenigstens einem im Wesentlichen komplementären Primer in einem Replikationsverfahren oder mit wenigstens zwei im Wesentlichen komplementären Primern in einem Amplifikationsverfahren, wobei der wenigstens eine im Wesentlichen komplementäre Primer an wenigstens ein Substrat gebunden wird; und

(c) Durchführen einer Rollender-Ring-Replikation an der wenigstens einen Hantelmatrize, die mit dem wenigstens einen im Wesentlichen komplementären Primer in Berührung steht, um wenigstens eine replizierte oder wenigstens eine amplifizierte Hantelmatrize auszubilden.

2. Verfahren nach Anspruch 1, wobei das doppelsträngige Nukleinsäuremolekül eine Länge von über 10 kb aufweist.

3. Verfahren nach Anspruch 1, wobei das doppelsträngige Nukleinsäuremolekül wenigstens 20 Kilobasen lang ist.

4. Verfahren nach einem der Ansprüche 1 bis 3, wobei:
das Verfahren vor dem Ligierungsschritt ferner ein Fragmentieren des wenigstens einen Nukleinsäuremoleküls umfasst, um wenigstens ein fragmentiertes Nukleinsäuremolekül mit einer Mindestlänge von über 5 kb, über 10 kb oder wenigstens 20 Kilobasen lang auszubilden.

5. Verfahren nach einem der Ansprüche 1 bis 3, wobei:
das Verfahren vor dem Berührungsschritt ferner ein Reinigen der wenigstens einen Hantelmatrize durch Behandeln einer beliebigen nicht ligierten Haarnadelstruktur und eines beliebigen nicht ligierten Nukleinsäuremoleküls mit einer Exonuklease umfasst.

6. Verfahren zum Nachweisen wenigstens einer replizierten oder wenigstens einer amplifizierten Hantelmatrize, wobei das Verfahren umfasst:

(a) Fragmentieren wenigstens eines Nukleinsäuremoleküls, um wenigstens ein fragmentiertes Nukleinsäuremolekül mit einer Mindestlänge von über 5 kb, über 10 kb oder wenigstens 20 Kilobasen lang auszubilden;

(b) Replizieren oder Amplifizieren des fragmentierten Nukleinsäuremoleküls gemäß dem Replikationsverfahren nach Anspruch 1; und

(c) Nachweisen der wenigstens einen replizierten oder der wenigstens einen amplifizierten Hantelmatrize.

7. Verfahren nach Anspruch 6, wobei der Schritt des Nachweisens der wenigstens einen replizierten oder der wenigstens einen amplifizierten Hantelmatrize darin besteht, die wenigstens eine replizierte oder die wenigstens eine amplifizierte Hantelmatrize zu sequenzieren.

8. Verfahren nach Anspruch 6, wobei der Schritt des Nachweisens der wenigstens einen replizierten oder der wenigstens einen amplifizierten Hantelmatrize das Inberührungbringen der wenigstens einen replizierten oder der wenigstens einen amplifizierten Hantelmatrize mit einer DNA-Sonde umfasst.

9. Verfahren nach Anspruch 6, wobei:
das Verfahren vor dem Berührungsschritt ferner das Reinigen der wenigstens einen Hantelmatrize durch Behandeln einer beliebigen nicht ligierten Haarnadelstruktur und eines beliebigen nicht ligierten fragmentierten Nukleinsäuremoleküls mit einer Exonuklease umfasst.

## Revendications

1. Procédé de réplication ou d'amplification d'au moins une molécule d'acide nucléique, le procédé comprenant :

(a) la ligature de structures en épingle à cheveux à chaque extrémité d'au moins une molécule d'acide nucléique double brin d'une longueur minimale supérieure à 5 kb isolée d'un échantillon pour former au moins une matrice en haltère ;

(b) la mise en contact de ladite au moins une matrice en haltère avec au moins une amorce sensiblement complémentaire dans un procédé de réplication ou avec au moins deux amorces sensiblement complémentaires dans un procédé d'amplification, ladite au moins une amorce sensiblement complémentaire étant fixée à au moins un substrat ; et

(c) le fait d'effectuer une réplication en cercle roulant sur ladite au moins une matrice en haltère mise en contact avec l'au moins une amorce sensiblement complémentaire pour former au moins une matrice en haltère répliquée ou amplifiée.

2. Procédé selon la revendication 1, dans lequel la molécule d'acide nucléique double brin a une longueur supérieure à 10 kb.

3. Procédé selon la revendication 1, dans lequel la molécule d'acide nucléique double brin a une longueur d'au moins 20 kilobases.

4. Procédé selon l'une quelconque des revendications 1 à 3, dans lequel :
avant l'étape de ligature, le procédé comprend en outre la fragmentation de l'au moins une molécule d'acide nucléique pour former au moins une molécule d'acide nucléique fragmentée ayant une longueur minimale supérieure à 5 kb, supérieure à 10 kb ou d'une longueur d'au moins 20 kilobases.

5. Procédé d'amplification selon l'une des revendications 1 à 3, dans lequel :
avant l'étape de mise en contact, le procédé comprend en outre la purification de ladite au moins une matrice en haltère en traitant toute structure en épingle à cheveux non ligaturée et toute molécule d'acide nucléique non ligaturée avec une exonucléase.

6. Procédé de détection d'au moins une matrice en haltère répliquée ou amplifiée, le procédé comprenant :

(a) la fragmentation d'au moins une molécule d'acide nucléique pour former au moins une molécule d'acide nucléique fragmentée ayant une longueur minimale supérieure à 5 kb, supérieure à 10 kb ou d'une longueur d'au moins 20 kilobases ;
(b) la réplication ou l'amplification de la molécule d'acide nucléique fragmentée selon le procédé de réplication selon la revendication 1 ; et
(c) la détection de ladite au moins une matrice en haltère répliquée ou amplifiée.

7. Procédé selon la revendication 6, dans lequel l'étape de détection de ladite au moins une matrice en haltère répliquée ou amplifiée consiste à séquencer ladite au moins une matrice en haltère répliquée ou amplifiée.

8. Procédé selon la revendication 6, dans lequel l'étape de détection de ladite au moins une matrice en haltère répliquée ou amplifiée comprend la mise en contact de ladite au moins une matrice en haltère répliquée ou amplifiée avec une sonde d'ADN.

9. Procédé selon la revendication 6, dans lequel :
avant l'étape de mise en contact, le procédé comprend en outre la purification de ladite au moins une matrice en haltère en traitant toute structure en épingle à cheveux non ligaturée et toute molécule d'acide nucléique fragmentée non ligaturée avec une exonucléase.

FIG.1

FIG.2

FIG.3

FIG.4

FIG.5

FIG.6

FIG.7

FIG.8

FIG.9

FIG.10

# FIG.11A

# FIG.11B

**EP 3 129 505 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2003108902 A1
- US 2011269193 A1
- US 2002012930 A1